# EUROPEAN PATENT SPECIFICATION

(11) **EP 0 487 709 B1**
(45) Date of publication and mention of the grant of the patent: **13.03.1996**
(21) Application number: 91912625.0
(22) Date of filing: 12.06.1991
(51) Int. Cl.: C12Q 1/68, C07H 21/04, C12P 19/34

(54) **DNA PROBES AND PRIMERS FOR DETECTION OF B. BURGDORFERI USING THE POLYMERASE CHAIN REACTION**
DNS-SONDEN UND PRIMER ZUR ERKENNUNG VON B. BURGDORFERI UNTER VERWENDUNG DER POLYMERASE-KETTENREAKTION
SONDES D'ADN ET AMORCES POUR LA DETECTION DE B. BURGDORFERI AU MOYEN DE L'AMPLIFICATION ENZYMATIQUE DU GENOME

(30) Priority: 15.06.1990 US 538957; 25.04.1991 US 691188
(43) Date of publication of application: 03.06.1992
(73) Proprietor: DADE INTERNATIONAL INC., Deerfield, Illinois 60015-0778 (US)
(72) Inventor: PICKEN, Roger, N., Schaumburg, IL 60173 (US); AMMONS, Harryl, C., Waukegan, IL 60085 (US)
(74) Representative: Bassett, Richard Simon
(86) International application number: US9104190
(87) International publication number: WO9119814

(56) References cited:
- WO-A-91/09870
- NUCLEIC ACIDS RESEARCH, vol. 17, no. 9, May 1989, IRL Press; G.S. GASSMANN et al., p. 3590/
- INFECTION & IMMUNITY, vol. 58, no. 6, June 1990, American Society for Microbiology; R. WALLICH et al., pp. 1711-1719/
- JOURNAL OF CLINICAL MICROBIOLOGY, vol. 28, no. 6, June 1990, American Society for Microbiology; D.C. MALLOY et al., pp. 1089-1093/
- JOURNAL OF INFECTIOUS DISEASES, vol. 160, no. 6, December 1989; P.A. ROSA et al., pp. 1018-1029/

## Description

### FIELD OF THE INVENTION

The present invention relates to nucleic acid probe assays for the detection of Borrelia burgdorferi or Borrelia hermsii present in biological samples in minute amounts.

### BACKGROUND OF THE INVENTION

Lyme disease is a complex of clinical disorders caused by the tick-borne spirochete Borrelia burgdorferi. It is the most common arthropod-borne disease in North America and Europe. Distinctive forms of the infection include erythema chronicum migrans, acrodermatitis chronica atrophicans, lymphocytic meningoradiculitis, and Lyme arthritis. Serious nervous system or cardiac involvement is observed in the second and third stages of the disease. Variations in clinical presentation and the disease's well-known mimicry of other neurological and rheumatological disorders make definitive diagnosis difficult. For a general review of the clinical and etiological aspects of Lyme disease, see Steere, et al., N. Engl. J. Med. 308:733 and Duray, Rev. Infect. Dis. 112:S1487. In spite of the extremely serious clinical course of Lyme disease, it responds very well to antibiotic treatment, if diagnosis and proper treatment can be instituted early. Early diagnosis, however, is frustrated by the unavailability of tests capable of reliable differential diagnosis within the first days or weeks post-infection. The diagnostic tests in current use are all serologic, and include indirect immunofluorescence (IFA), enzyme-linked immunosorbent assay (ELISA)and immunoblotting. Such immunologic tests are unreliable because only about 50-60 percent of patients with early disease have diagnostic titers measurable by either IFA or ELISA during the first three to six weeks post-infection.

In addition, false positive reactions in IFA and ELISA have been reported in patients having syphilis caused by T. pallidum, and relapsing fever caused by Borrelia hermsii. The prevalence of false positives in serologic based assays for B. burgdorferi is attributable to the high degree of genetic interrelationship amongst pathogenic spirochetes resulting in serological cross-reactivity of the major structural determinants. See, for example, Magnarelli, et al., J. Infect. Dis., 156:183 (1987).

An alternative to serologic assays is a test based upon the property of nucleic acid probes to bind very specific target sequences. Since in Lyme disease the organism itself is present during infection in very low numbers, it is necessary to first amplify the target nucleic acid sequences so that the probes to which a signal generating molecule is attached, hybridize in sufficient number to generate a detectable signal. The strategy of such probe based assays is that the target sequence be specific for the species of organism sought to be detected, and recognition of target sequences encompass every member strain of the species.

A nucleic acid probe assay for B. burgdorferi must be able to distinguish between this organism and closely related species, in particular variants of relapsing fever, B. hermsii, B. parkeri, B. turicatae, B. hispanica, and B. recurrentis. DNA homology studies on a variety of Borrelia indicate varying degrees of homology, with B. hermsii showing the greatest homology at 58 to 63 percent, as reported in Hyde and Johnson, Zbl. Bakt. Hyg. A, 263:119 (1988). Three North American species, B. hermsii, B. turicatae and B. parkeri share 77 to 100 percent homology, but comparison of European and North American strains indicates substantial intra-species variation.

In the detection of B. burgdorferi by nucleic acid probe assays, attention has focused on probes for the OspA and OspB surface protein antigens. Nielsen, et al., Mol. Cel. Probes, 4:73 (1990) reported successful probing of an amplified 145 base pair segment of OspA to detect as little as 50 fg of B. burgdorferi DNA corresponding to 50 spirochetes. The probe was found to be specific for B. burgdorferi in that no cross-reactivity was observed for B. hermsii, T. pallidum, T. denticola, or S. aureus. However, Persing, et al., J. Clin. Micro., 28:566 (1990) probed the OspA region of a number of bonafide B. Burgdorferi isolates, and found that the probes were unable to positively identify all of the isolates. The molecular basis of this result was the finding that some B. burgdorferi strains possess an extensively different OspA protein or lack the OspA protein altogether, and possess instead a smaller protein termed pC. Hence, at present there is no definitive assay for B. burgdorferi.

More recent studies indicate that B. burgdorferi may comprise more than one group of organisms. Postic et al., Res. Microbiol., 141: 465 (1990) analyzed thirteen strains from diverse sources by DNA/DNA hybridization, and found that a subgroup of four strains could be distinguished from nine reference strains. Rosa, et al. J. Clin. Microbiol., 29: 524 (1991) also found that two subgroups of B. burgdorferi could be identified by DNA amplification patterns using a B. burgdorferi specific target sequence from an unknown portion of the genome. It should be noted that there were major discrepancies between the results of these two groups, indicating that these systems may not have satisfactorily specific diagnostic value.

### SUMMARY OF THE INVENTION

Comparison of the DNA sequences encoding the highly conserved flagellin genes from B. burgdorferi and B. hermsii reveal a small region of relative non-homology containing 50 to 70 percent mismatched bases including short deletions. Primers selected from this region were found to direct B. burgdorferi specific amplification of the interprimer sequence. Probes constructed for the interprimer region specifically identify B. burgdorferi.

In accordance with the present invention, a bioassay utilizing such primers and probes for specific detection of B. burgdorferi in a biological specimen comprises
extracting the nucleic acid fraction from the specimen,
adding primers of complementary strand specificity to sequences flanking a semiconserved region of nucleic acid encoding the flagellar open reading frame
hybridizing the primers to the complementary strand sequences
amplifying the semiconserved region by nucleic acid amplification techniques
hybridizing the amplified semiconserved region to a signal-generating probe constructed from the interprimer sequence, and
detecting the signal emitted by the hybridized probe.

In another aspect of the present invention, oligonucleotide primer pairs are selected for specificity to B. burgdorferi in the flagellar open reading frame region, a first such primer comprising a sequence of about 8-32 consecutive bases from nucleotide numbers 390 to 712 of the flagellar open reading frame, and a second such oligonucleotide primer comprising a sequence on the complementary strand comprising 8-32 substantially consecutive bases having not greater than 70 percent homology with the corresponding sequence of B. hermsii.

It is preferred if the second oligonucleotide is located spacedly at least 35 nucleotides from the 5' end of the first said primer.

A further aspect of the invention involves the selection of probes for specific detection of nucleic acids amplified from the non-homologous region of the flagellin gene open reading frame. These probes comprise signal-generating oligonucleotide tracers substantially homologous to a sequence of the flagellar open reading frame of B. burgdorferi, but only about 70 percent homologous to the corresponding sequence of B. hermsii. Signal-generating means conjugated to the tracers may be radiolable, a fluorescent label, or a biotinylated label.

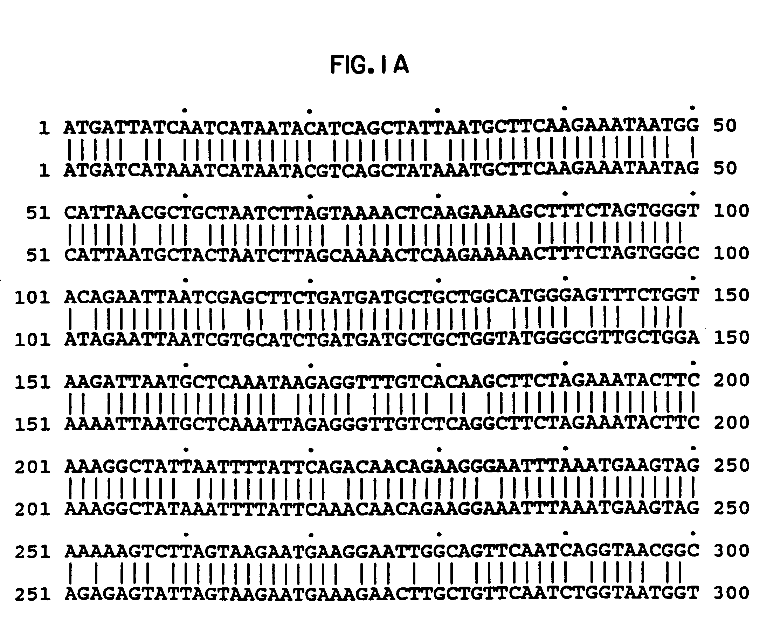

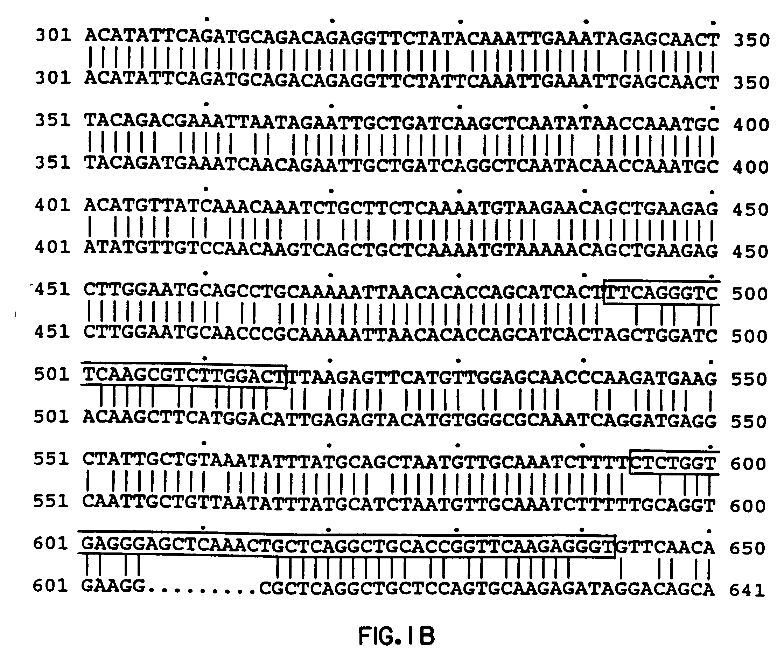

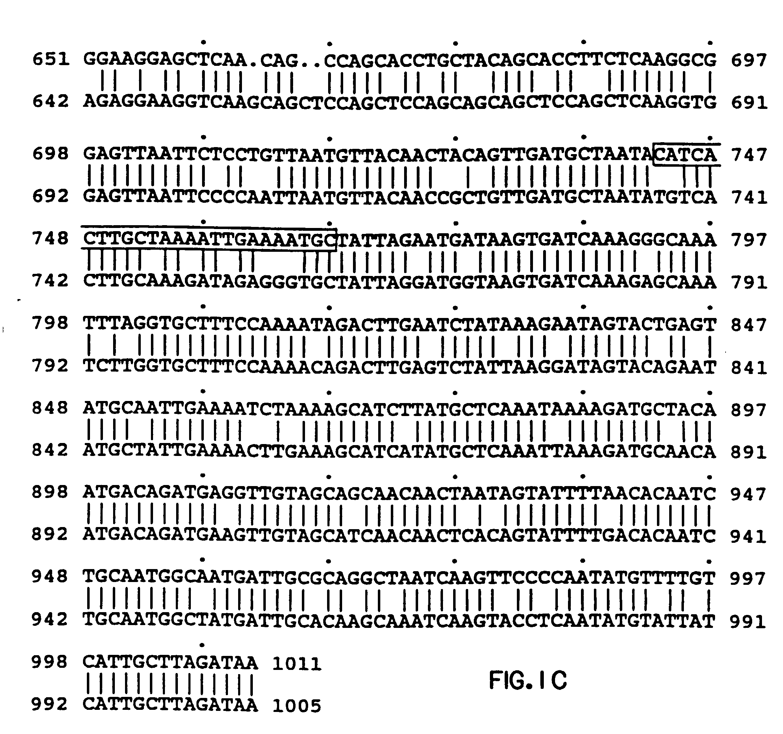

The said B. burgdorferi flagellin gene open reading frame is substantially as described in Figure 1 or is wherein the said flagellin gene is substantially the same as the flagellin gene of B. burgdorferi strain B31 ATCC 35210.

The said corresponding B. hermsii sequence is substantially as described in Figure 1 or wherein the said corresponding sequence is substantially the same as the sequence of the flagellin gene of B. hermsii ATCC 35209.

A probe containing an oligonucleotide having the sequence CTC TGG TGA GGG AGC TCA AAC TGC TCA GGC TGC ACC GGT TCA AGA GGG T, was used to screen a number of B. burgdorferi strains from which amplifications of the interprimer sequence had been carried out. The probe detected some amplified nucleic acids, but not others. Accordingly, it is a further aspect of the present invention to utilize the sequences of these interprimer regions as targets for detection of subgroups of B. burgdorferi. One variant sequence of the B. burgdorferi flagellin gene open reading frame hypervariable region extending from nucleotide number 517 to number 742 comprises base substitutions of a guanosine at positions 531, 549, 552, 612, 613, 684, 693, adenosine at positions 539, 591, 603, 622, 639, 651, 666, thymidine at positions 540, 573, 630, 696, and cytosine at position 735. Another variant sequence of the B. burgdorferi flagellin gene open reading frame hypervariable region extending from nucleotide number 517 to number 724 comprises base substitutions of a guanosine at positions 531, 595, 612, 649, and 663, adenosine at positions 539, 552, 651, 666, 670, and 688, thymidine at positions 540, 571, 594, 630, and 696, cytosine at positions 644 and 726. Finally, a set of probes complementary to a portion of the target sequences comprise an oligonucleotide tracer for detection of subgroups of B. burgdorferi comprising an oligonucleotide probe having a sequence selected from the group consisting of CTC TGG TGA GGG AGC TCA AAC TGC TCA GGC TGC ACC GGT TCA AGA GGG T, CTC TGG TGA AGG AGC TCA GGC TGC TCA GAC TGC ACC TGT TCA AGA AGG, and TGC TGG TGA GGG AGC TCA AGC TGC TCA GGC TGC ACC TGT TCA AGA GGG TGC, and signal means conjugated to said probe.

### DETAILED DISCLOSURE OF THE PREFERRED EMBODIMENT

In prior attempts to develop a probe based assay for amplified target sequences of B. burgdorferi, the major surface antigen proteins expressed from the OspA and OspB genes were selected. Since not all bonafide B. burgdorferi strains are found to encode these antigens, it is apparent that a different sequence must be selected.

In spirochetes, the endoflagellum is composed of an assemblage comprising a single subunit. Wilske, et al., Zbl. Bakt. Hyg. A 263:92 (1986) reported that monoclonal antibodies isolated against the flagellin protein of B. burgdorferi also reacted with B. hermsii, B. duttonii, B. turicatae and B. parkeri. Because of its unitary subunit structure, evidence of antigenic homology, and the combination of both structural and physiological constraints within the same molecule, the flagellin gene may be highly conserved, and therefore be likely, as a target in a probe based assay, to identify every member of the species. Cross-reaction of several species to the same antisera, however, also suggest that these genes may be highly conserved between species in the same genus.

Isolation and sequencing of the flagellin gene for B. hermsii, the most closely related species to B. burgdorferi, in fact, revealed large regions of homology with B. burgdorferi. Unexpectedly, however, there was also a region of approximately 480 base pairs within the open reading frame of the flagellin gene which contains sequences of relative non-homology. In the present invention, these sequences of relative non-homology provide the basis for constructing primers and probes which are specific in amplifying and detecting B. burgdorferi, but which distinguish other closely related species.

Figure 1 shows the homology comparison of the nucleotide sequences of the flagellin genes from B. burgdorferi and B., hermsii. Alignment of the nucleotides was assisted by the UWGCGT analysis program BESTFIT, as referenced in Nuc. Acids Res., 12:126 (1988). The B. burgdorferi sequence is positioned above the B. hermsii sequence. It is apparent from the comparison that the region of non-homology is bracketed by nucleotides at approximately positions 390 and 712. The region from about nucleotide position 475 to 770 are preferred for primer and probe construction because it contains at least two small deletions in the B. hermsii sequence.

In selecting primer sequences, it is desirable to utilize oligonucleotides for preferably about 8 to 32 nucleotides. Use of less than 8 nucleotides reduces specificity and decreases binding affinity. Use of greater than about 32 nucleotides does not enhance the priming function or increase specificity, and may increase the probability of binding in regions of significant mismatch. However, there will be sequences within the non-homologous region in which a primer of 6 or 7 nucleotides, or greater than 32 would be operable, and are to be considered the equivalents of the preferred sequence length.

The strategy in primer selection is to identify short sequences native to B. burgdorferi which are maximally non-homologous to the corresponding sequences of B. hermsii. In the amplification reaction, the primers Will anneal only to the B. burgdorferi template and not to B. hermsii DNA, so that no specific amplification of sequences hybridizable to the signal-generating probe will occur. Additionally, primers are selected so as to flank the target region on opposite strands. The target region should be at least as long as the probe to which it hybridizes, preferably about 50 nucleotides in length. Thus primer pairs are selected which comprise a first oligonucleotide selected from the group consisting of a sequence comprising about 8-32 substantially consecutive bases from nucleotide numbers 390-712 of the flagellar gene open reading frame, and a second oligonucleotide selected from the group consisting of a sequence on the complementary strand located spacedly about 50 nucleotides from the 5' end of the first primer, comprising 8-32 substantially consecutive bases having not greater than 70 percent homology with the corresponding sequence of B. hermsii.

The selected primers are utilized as initiation points on the denatured nucleic acid template for chain extension on the complementary strand. Conveniently, the amplification of nucleic acids is carried out using the polymerase chain reaction (PCR), as described in U.S. Patent No.s 4,683,195 and 4,683,202 (Mullis). In this method, target DNA is denatured, and primers are hybridized to complementary sequences flanking the target region to be amplified on the respective opposite strands. DNA polymerase is added together with nucleotide triphosphates and new DNA is synthesized from the primer. When the reaction is complete, the DNA is once again denatured, more polymerase is added, and another round of DNA synthesis occurs. By repeating this a number of times, a high degree of amplification on the order of 5-6 logs can be achieved. It is desirable to conduct the hybridizations and polymerizations under as stringent a set for conditions as possible, that is, at elevated temperatures wherein the binding of primers requires even greater fidelity to the exact sequence. The preferred temperature is 65 degrees Centigrade, which requires use of a thermostable polymerase, such as that described in U.S. Patent No. 4,889,818 (Gelfand et al.).

Other protocols for nucleic acid amplification known in the art may be substituted for PCR. One such method, called 3SR, utilizes reverse transcriptase to create an RNA/DNA hybrid from which a duplex DNA structure containing a T7 or other suitable transcriptional promoter is produced. A DNA dependent RNA polymerase is added in the system and a large number of target sequence specific transcripts are synthesized. These amplified transcripts are then probed with a suitable signal generating oligonucleotide to detect the specific target. In constructing the primers for 3SR or a related amplification method called TAS, the promoter sequence must be conjugated to the 5' end of the primer. The T7 promoter having the sequence 5'-TAATACGACTCACTATAGGGA is preferred.

The probe sequence may be selected from any interprimer sequence in the non-homologous open reading frame region. The best probes are those incorporating a sequence of maximum mismatch. In this region, the sequence from about base number 585 to about base number 650 is especially efficacious because it spans the region containing the deletions and has an overall approximately 50 percent mismatch. In the preferred embodiment, selection of a probe rather than a primer or primers from this region is based on the observation that a highly mismatched probe will not hybridize significantly with target at low levels of amplification, thus resulting in a substantially qualitative assay.

The probe length may be varied over a potentially wide range from a few to hundreds of base pairs. However, a balance must be struck between the specificity of the probe and its stability. The shorter probes, particularly those selected from highly non-homologous regions, are more highly specific than longer ones, but tend to be unstable at temperatures that give good hybridizing stringency. Longer probes are less specific generally because they are able to tolerate a greater number of mismatches. The preferred probes are those selected from regions of relative non-homology, have relatively high GC content, and are about 35-55 nucleotides.

Utilizing an oligonucleotide selected from a region encompassed by nucleotide positions 594 to 642 of the flagellin gene open reading frame having the highest degree of mismatch and non-homology, namely, CTC TGG TGA GGG AGC TCA AAC TGC TCA GGC TGC ACC GGT TCA AGA GGG T, a ³P conjugated probe was constructed. A number of putative B. burgdorferi strains were screened by first amplifying the target region extending between the loci within the flagellin gene to which the above-described primers anneal (the interprimer amplification region).

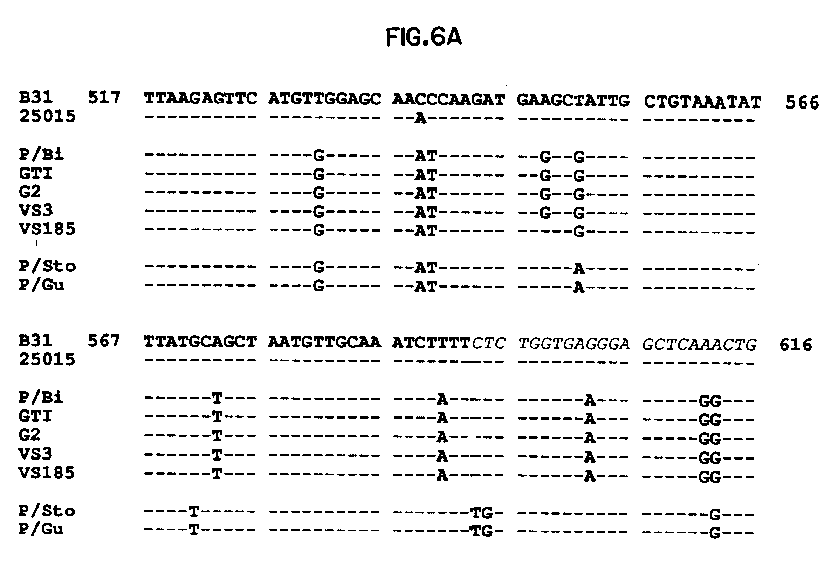

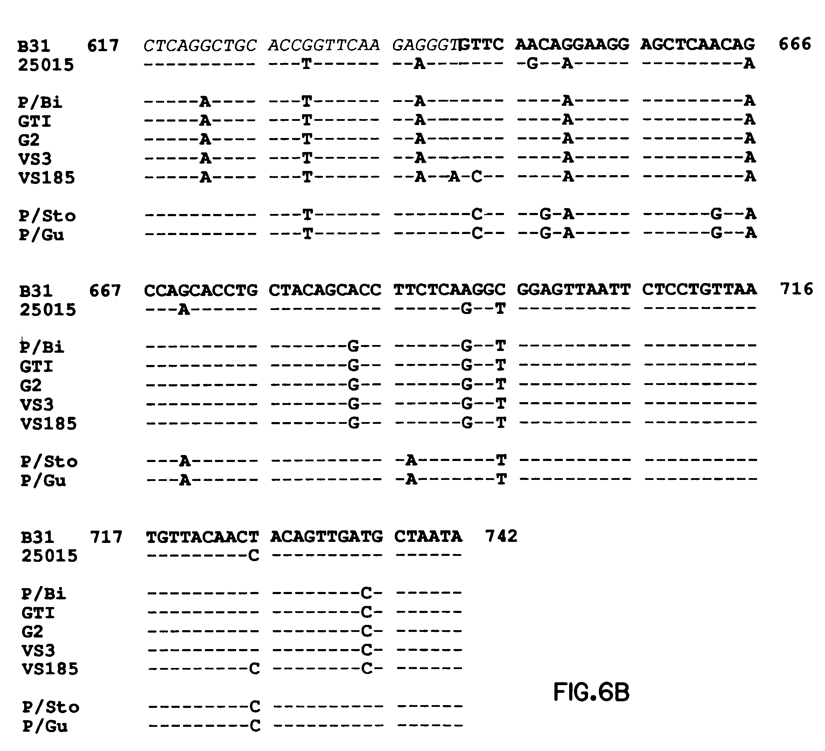

The amplified nucleic acids were then probed with the above probe in a Southern blot assay. Surprisingly, some of the bonafide B. burgdorferi strains showed hybridization to the probe, and others did not. The amplified regions were sequenced. The sequences are shown in Figure 6.

These sequences contain several positions of mismatched bases. By selecting a region containing minimally 5 mismatched bases, three probes were obtained which could distinguish three subgroups of B. burgdorferi in an assay in which the interprimer region was first amplified, and then the amplified probes were probed with the probes of specific sequence. The three probe sequences are as follows: Probe 1: CTC TGG TGA GGG AGC TCA AAC TGC TCA GGC TGC ACC GGT TCA AGA GGG T, Probe 2: CTC TGG TGA AGG AGC TCA GGC TGC TCA GAC TGC ACC TGT TCA AGA AGG, and Probe 3: TGC TGG TGA GGG AGC TCA AGC TGC TCA GGC TGC ACC TGT TCA AGA GGG TGC.

Table 1 gives the individual subgroups of B. burgdorferi strains which are specifically detected by each of the above corresponding probes. Table 2 give the sequences of the 3 subgroup probes.

Applicants have found that the above specific sequences are most preferred because of the stringency of the assay obtained where the extent of mismatched bases is maximized for substantially corresponding regions of the flagellin gene. However, it will be apparent that minor inconsequential base substitutions may be made which will not adversely affect the specificity of the assay. Such minor sequence variations are considered by Applicants to be the equivalents of the sequences disclosed herein.

The probes in molecular combination with signal generating means become oligonucleotide tracers capable of tagging the target sequence to

**Table 1**

| **BORRELIA SPECIES AND STRAINS TESTED** | | | | | | |
|---|---|---|---|---|---|---|
| SPECIES B31 SUBGROUP | STRAIN DESIGNATION | GEOGRAPHIC LOCATION | PRIMERS | PROBE 1 | PROBE 2 | PROBE 3 |
| B. burgdorferi | B31 [ATCC 35210] | New York | + | + | - | - |
| | IRS [ATCC 35211] | Switzerland | + | + | - | - |
| | Virulent MM1 | Minnesota | + | + | - | - |
| | Virulent MMT1 | Minnesota | + | + | - | - |
| | Virulent I.pacificus | California | + | + | - | - |
| | N40 | New York | + | + | - | - |
| | Son 328 | California | + | + | - | - |
| | DN-127 | California | + | + | - | - |
| | Minnesota Mouse | Minnesota | + | + | - | - |
| | Lake 339 | California | + | + | - | - |
| | France 20001 | France | + | + | - | - |
| | CD16 | Minnesota | + | + | - | - |
| | VS215 | Switzerland | + | + | - | - |
| | NE56 | Switzerland | + | + | - | - |
| | 25015 | New York | + | + | - | - |
| | P/Bi | Germany | + | - | - | + |
| | P/Sto | Germany | + | - | + | - |
| | P/Gu | Germany | + | - | + | - |
| | GTI | Germany | + | - | - | + |
| | G2 | Germany | + | - | - | + |
| | VS3 | Switzerland | + | - | - | + |
| | VS185 | Switzerland | + | - | - | + |
| B. hermsii | HS1[ATCC 35209] | | | | | |
| | Frogner | | | | | |
| | YOR-1 | California | - | - | - | - |
| | CON-1 | California | - | - | - | - |
| | MAN-1 | California | - | - | - | - |
| B. parkeri | | | - | - | - | - |
| B. turicatae | | | - | - | - | - |
| B. crocidurae | | | - | - | - | - |
| B. anserina | | | - | - | - | - |
| B. coriaceae | | | - | - | - | - |
| B. coriaceae | Co57[ATCC 43381] | | - | - | - | - |

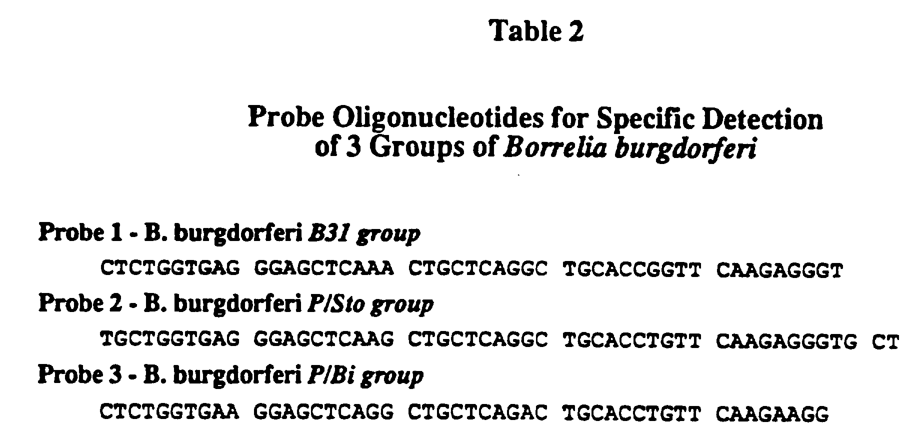

which they bind, or of emitting a signal either directly or indirectly. Such tracers may conveniently be a probe conjugated to a radioactive molecule or enzyme. Upon hybridizing of tracer to target, the double-stranded duplex may be separated from unhybridized tracer by conventional separation techniques, and the amount of bound radioactivity measured in a scintillation or gamma counter. Similar conventional separation techniques may be used to separate enzyme-linked hybridized tracers. Upon separation of the enzyme-conjugated duplex, a colorimetric or fluorometric substrate is added, and the signal detected by conventional instrumentation. Other signal-generating systems known in the art include chemiluminescence utilizing acridinium esters or dioxetanes, as described in McCapra, et al., Chemiluminescence and Bioluminescence, Plenum Press, N.Y. (1973), fluorescent tags such as fluorescence assays, or biotin-conjugated probes.

Of particular interest, is the application of fluorescence polarization (FP) which measures the increase in polarization resulting from hybridization of a fluorescein-tagged probe to its target. The importance of this detection system is that detection can proceed immediately upon annealing of tracer to target without first separating unbound tracer. This detection method is described in greater detail in Example 2 hereinbelow.

Utilizing the primers and probes of the present invention, bioassays are conveniently configured for the diagnostic detection of B. burgdorferi from biological specimens. In such bioassays, the best source of primers and probes is by conventional methods of oligonucleotide synthesis, although purified cloned gene fragments of proper sequence may also be employed. Biological specimens of interest include skin punch biopsies, blood and blood fractions, cerebrospinal fluid, the tick carrier and its body parts, synovial fluid, and fractions of body tissue implicated in the Lyme disease complex. Techniques for punch biopsy specimens are described in Berger, et al., J. Am. Acad. Dermatol., 13:444 (1986). Isolation of spirochetes from blood is described in Benach, et al., N. Eng. J. Med., 308:740 (1983). Isolation of spirochetes from cerebrospinal fluid is described in Karlsson, et al., J. Clin. Microbiol., 28:473 (1990). Isolations from other tissues are also described in the literature.

In order to carrying out the amplification step preliminary to the tracer hybridization, it is necessary to extract the nucleic acids to render them available. Extraction methods for obtaining nucleic acids from biological specimens are well-known in the art, and are deemed conventional in carrying out the inventive bioassays. For example, Keller & Manak, DNA Probes, Chapter 2, "Sample Preparation", offer complete details for a number of the standard methods including the original literature references. Further preparation of the nucleic acids by denaturation is also conventional, and may be effected either by manipulation of pH or salt concentration, or by heating.

The primers, which upon hybridization to the template sequences, act as substrates for polymerase-mediated chain extension, may be added to the reaction mix either before or after denaturation, and annealed upon shifting to renaturing conditions. The polymerase is then added, and successive cycles of chain extension allowed to occur. In PCR, DNAtaq polymerase is preferred. In 3SR, reverse transcriptase first creates and RNA/DNA hybrid which upon RNAse H digestion and repolymerization with DNAtaq polymerase can be repetitively transcribed with DNA dependent RNA polymerase. Methods of detecting the probed amplified nucleic acids have already been described hereinabove.

A particularly powerful method of detecting the hybridized radioactive tracer subjects the products of nucleic acid amplification to agarose electrophoresis, with transfer of the nucleic acid bands to a nylon membrane such as Pall® Biodyne® B, followed by hybridization to the signal-generating probe, in a standard Southern blot assay [Southern, J. Mol. Biol., 98:503 (1975)]. The areas of specific hybridization are then visualized by autoradiography of the probed nucleic acid.

Thus, in the bioassays of the present invention, a biological specimen containing suspected sequences of B. burgdorferi nucleic acids is nucleic acid extracted and denatured in the presence of primers of complementary strand specificity to sequences flanking a semiconserved region of nucleic acid encoding the flagellar open reading frame, hybridizing the primers to the complementary strand sequences, amplifying the semiconserved regions by nucleic acid amplification techniques, hybridizing the amplified semiconserved region to an oligonucleotide tracer having a sequence substantially homologous to a sequence of the flagellar gene open reading frame of B. burgdorferi but maximally 70 percent homologous to the corresponding sequence of B. hermsii, and detecting s signal emitted by signal generating means of the tracer

Further advantages of the present invention will be apparent from the Example which follows:

### EXAMPLE 1

B. hermsii and B. burgdordferi were obtained from the American Type Culture Collection under accession numbers ATCC 35209 and ATCC 35210 respectively. The cells were grown up and harvested, and the DNA extracted by conventional methods. The chromosomal DNA was partially digested with Sau 3A restriction enzyme, and the fragments characterized on agarose gel electrophoresis. Suitable sized fragments of 9-23 bp were partially filled in the presence of dGTP and dATP. This material was then readily cloned into lambda GEM11 vector, which had been digested with Xho I restriction nuclease and partially filled in with dTTP and dCTP.

Lambda libraries were then plated using E. coli LE392 as host. Plates were plaque-lifted using Pall® Biodyne® B membranes, and screened by hybridization as in Souther blot analysis. Synthetic primers prepared at random from the known sequence of B. burgdorferi, as reported by Gassman, et al., Nuc. Acid Res., 17:3590 (1989), were utilized to amplify templates derived from B. burgdorferi and B. hermsii. In one instance a primer pair utilized to generate a 300bp fragment from B. burgdorferi quite unexpectedly resulted in a faint band of 300 bp when used to amplify B. hermsii. The faint bank identified a slight amplification of a region within the flagellin gene which was otherwise relatively non-homologous to B. burgdorferi. Utilizing this fragment as a probe permitted identification of lambda clones containing this portion of the gene. Construction of restriction maps from these clones and sequencing of the constructs, gave the correct sequence for the non-homologous sequence shown in Figure 1.

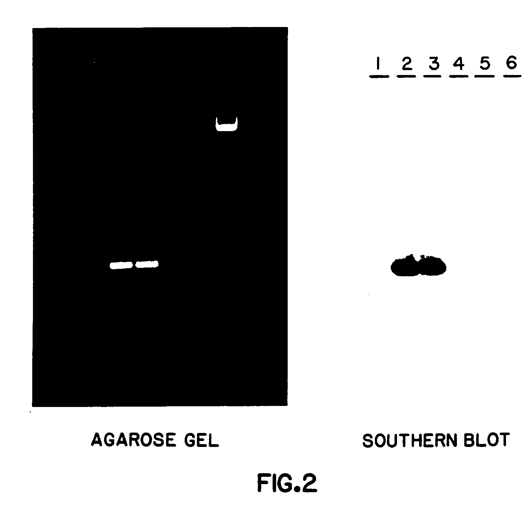

In a typical experiment, utilizing the primers indicated in Figure 1, amplification of the interprimer region was performed by thirty cycles of PCR. The amplification products were then analyzed as follows: electrophoresed in agarose gel followed by Southern blot analysis. Referring to Figure 2, the left panel shows an agarose gel electrophoresis pattern. The two bands visible together at lower center correspond to the amplification products of two independent strains of B. burgdorferi, including a strain negative in amplification studies utilizing amplification of the OspA gene. It is apparent that the molecular size in each case corresponds to the expected size for amplification of the correct targeted region. The controls are all negative. To confirm that the small bands visible at other segments of the gel for the controls represented nonspecific amplification, the gel was subjected to Southern blot analysis, as shown in the right panel of Figure 2. The transferred nucleic acids were probed with a tracer containing the sequence shown in Figure 1. The results confirm the specificity of the assay.

In a second experiment, the amplified B. burgdorferi and control DNA were analyzed in homogeneous assay by hybridization with a probe, fluorescein conjugated at its 5' end having a sequence: 5'GAGCTCCCTCACCAGAGAAA, by fluorescence polarization. The probe mix contained 1.4 ml of SSPE buffer [Sambrook, et al., Molecular Cloning: a laboratory Manual, 2 Ed., Cold Spring Harbor Laboratory, (1989)], 7 ul of 3.2 M Tris-HCl, 250 fmol probe conjugate, and 200 ul formamide. In a separate tube, a sample of the PCR amplified material was denatured in 0.1 M NaOH for 15 minutes at 55 degrees Centigrade. The sample was then added to the probe mix and fluorescence polarization readings were taken over a 72 minute time period with a Fluorescence Polarimeter.

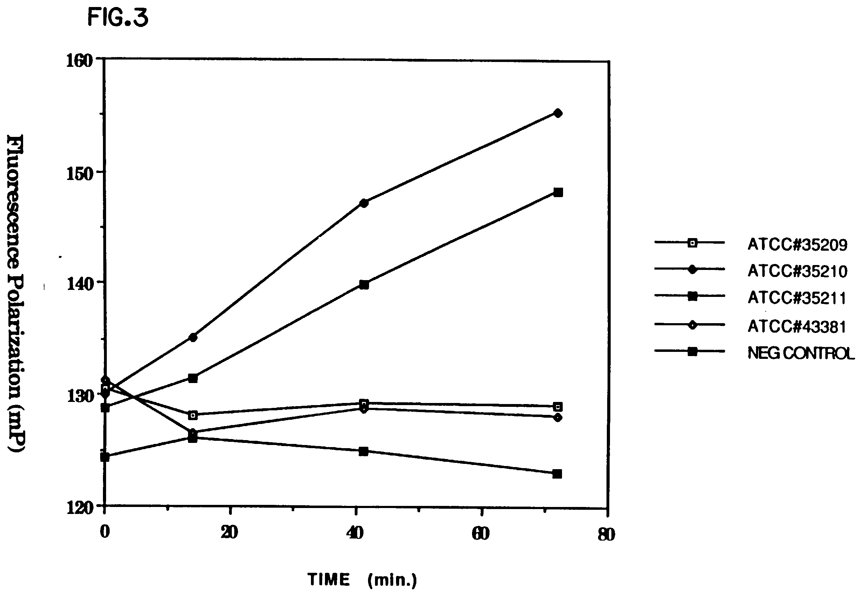

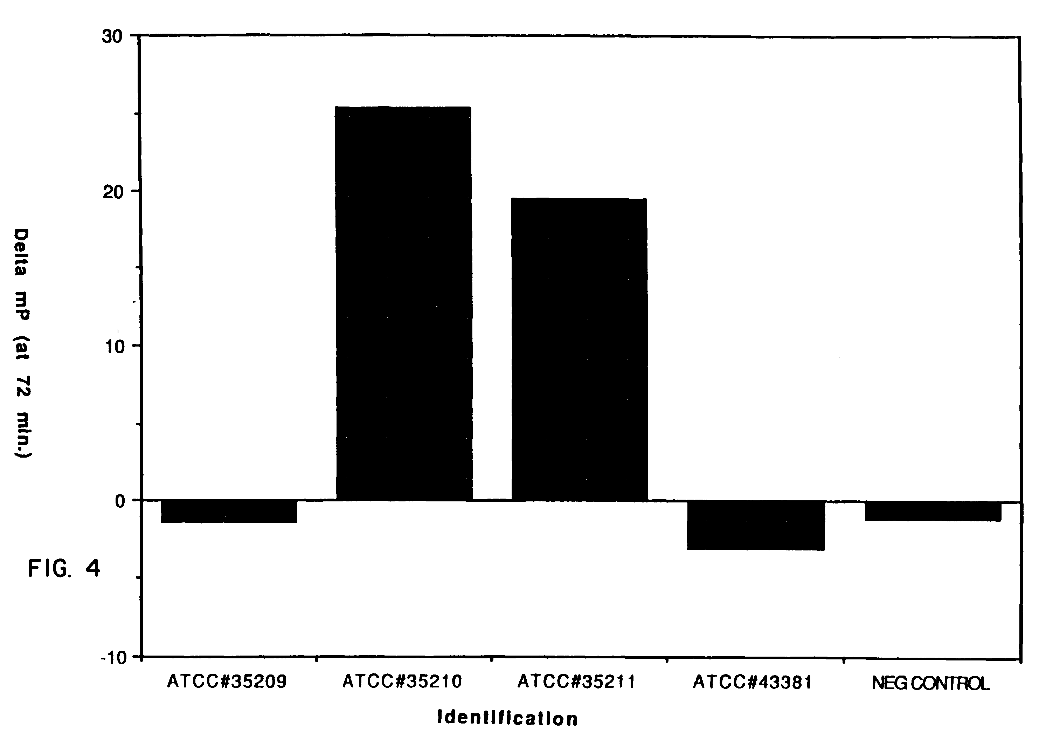

The results are graphed in Figure 3, and show that specific polarization is qualitatively detected for B. burgdorferi samples (designated ATCC#35210 and ATCC#35211 respectively). The controls are negative (ATCC#35209, B. hermsii; ATCC#43381, B. coriaceae). Referring to Figure 4, which shows a barograph of the difference in MP (millipolarization units) between the value at 72 minutes and at substantially time zero, it is clear that FP is capable of specifically identifying an amplified target sequence in a homogenous format.

### EXAMPLE 2

DNA from the series of B. burgdorferi strains listed in Table 1 was extracted. Amplification by polymerase chain reaction (PCR) was carried out as described above, utilizing the primers set forth in Example 1. Subgroup 1 of B. burgdorferi, as shown in Table 1 is designated B31. Subgroup 2 is designated P/Bi, and subgroup 3 is designated P/Sto. Probes 1-3, having the sequences shown above, are specific for the Subgroups B31, P/Sto, and P/Bi respectively.

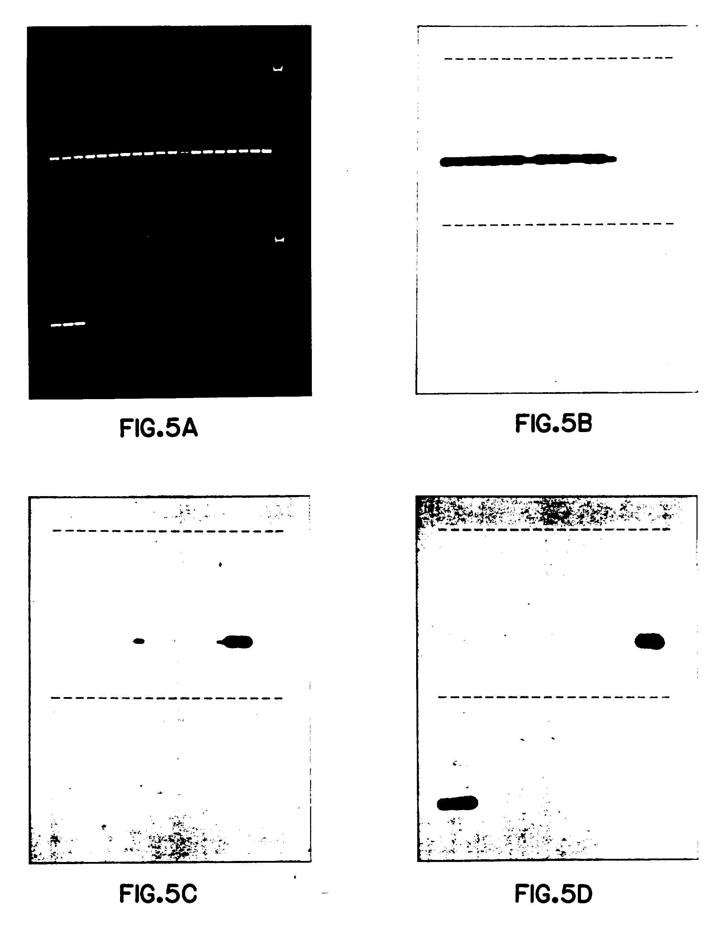

Hybridizations were carried out and the hybridization products were anlyzed by gel electrophoresis, according to the methods set forth in Example 1. Figure 5A shows the bands corresponding to each electrophesed amplified sample. The gel bands were then Southern blotted onto membranes and probed with ³P labelled probes 1-3. The membranes were washed twice for 30 minutes in 2X SSC buffer followed by two 30 minute washed in 0.2X SSC buffer. Wash temperatures were 67.3 degrees C (B31 group probe), 68.3 degrees C (P/Sto group probe), and 65.8 degrees C (P/Bi group probe). Further details of the Southern blot procedure are set forth in Picken, BioTechnique, 9:412 (1990). The results, shown in Figures 5B, C, and D, indicate that each probe is completely specific for its corresponding subgroup of B. burgdorferi indicated in Table 1.

### EXAMPLE 3

In a further series of experiments, DNA from a panel of Borrelia was amplified using primers of complementary strand specificity flanking a sequence of B. hermsii in corresponding position to those described for B. burgdorferi. The first oligonucleotide primer was selected from the group consisting of sequences comprising about 8-32 substantially consecutive bases from nucleotide numbers 390 to 770 of the flagellin gene open reading frame. A second oligonucleotide primer was selected from the group consisting of sequences on the complementary strand located spacedly about 35 to 55 nucleotides from the 5' end of the first primer, comprising about 8-32 nucleotides having not greater than 70 percent homology with corresponding sequence of H. burgdorferi.

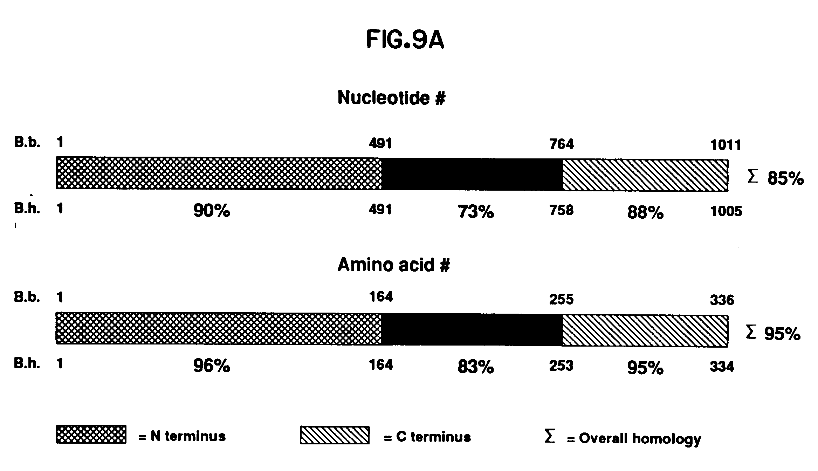

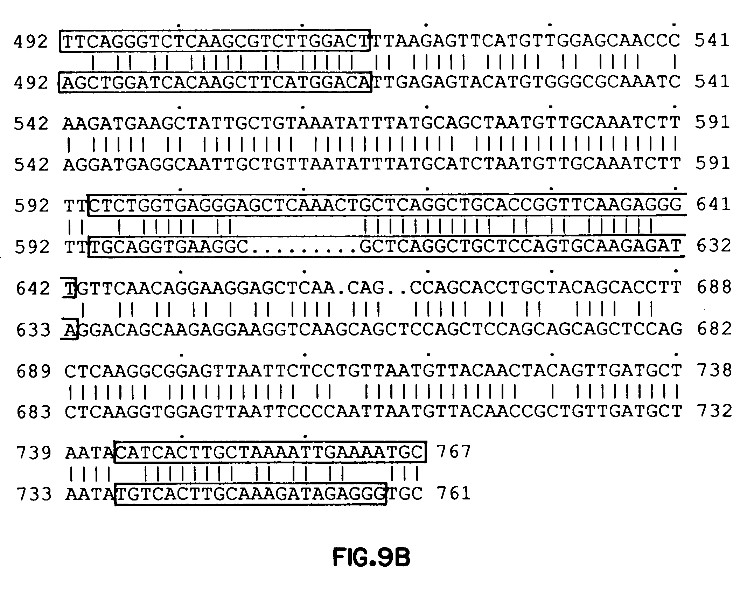

The primers utilized in these experiments are shown in Figure 9.

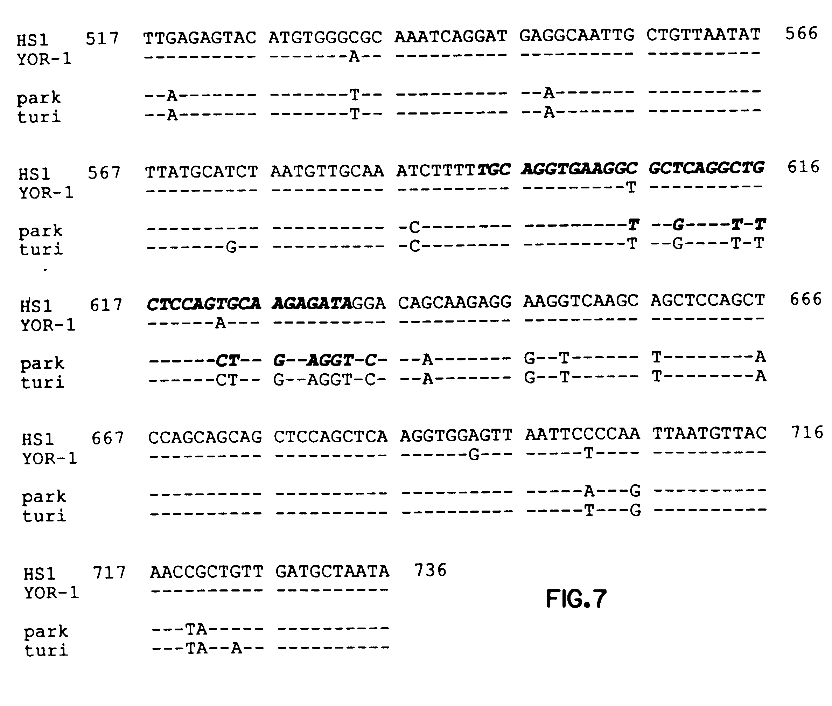

It was found that these primers amplified all the B. hermsii strains tested in addition to B. parkeri and B. turicatae. Upon sequencing the amplified DNA of the B. parkeri and B. turicatae, differences in the inteprimer sequence were noted for these organisims compared to B. hermsii. These differences are shown in Figure 7. A probe was constructed selecting the sequence shown in Figure 7 in bold letters. After gel electrophoresis, the amplified bands were transferred to a membrane and Southern blotted utilizing the probe. All procedures were as given in the preceding examples, except that the wash temperatures for the B. hermsii probe was 57.7 degrees C., and the wash temperature for the B. parkeri/turicatae probe (shown in Figure 7 as the bold letters of B. hermsii (HS1) with the bold substitutions at the nucleotide positions in which those strains (park and tri) differ from HS1) was 62.0 degrees C.

The probe sequence contains essentially a core sequence from nucleotide number 606 to 633, but bases may be added at either end, to stabilize *hybrid formation*, or to adjust the stringency of the wash procedures. The prefered probe thus comprises an oligonucleotide having the sequence TGC AGG TGA AGG CGC TCA GGC TGC T CCA GTG CAA GAG ATA conjugated to signal generating means such as_{32p.}

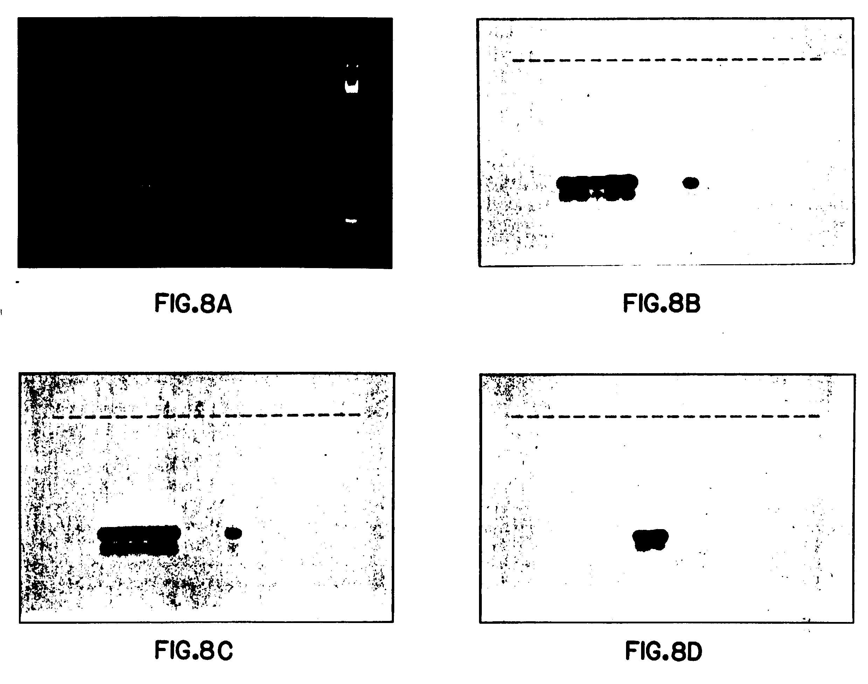

Utilizing a probe having the nucleotide sequence of B. hermsii shown in bold letters in Figure 7, permitted the differential identification of B. hermsii. The Southern blot analysis shown in Figure 8 confirms the high specificity of the probe. This has clinical significance in that B. hermsii, which causes relapsing fever can be distinguished from the causative agent of Lyme Disease, and from Borrelia organisms causing animal disease.

### INDEX TO LEGEND FOR FIGURE 5

The order of strains from left to right in each of the gel pictures 5A-5D is listed below; 1-20 refer to the top row and 21-40 refer to the bottom row.

### Borrelia burgdorferi

- 1.*: B31 [ATCC #35210)
- 2.: IRS [ATCC #35211]
- 3.: Virulent MM1
- 4.: Virulent MMT1
- 5.: Virulent I.pacificus
- 6.: N40
- 7.: Son 328
- 8.: DN127
- 9.: Minnesota Mouse
- 10.: Lake 339
- 11.: France 20001
- 12.: CD16
- 13.: VS215
- 14.: NE56
- 15.: Millbrook 25015
- 16.: P/Sto
- 17.: P/Gu
- 18.: P/Bi
- 19.: German Tick Isolate
- 20.: 123 Base Pair Ladder M. Wt. Marker
- 21.: G2
- 22.: VS3
- 23.: VS185

* Gel lane number

### Borrelia hermsii

- 24.: HS1 [ATCC #35209]
- 25.: Frogner
- 26.: YOR-1
- 27.: CON-1
- 28.: MAN-1
- 29.: *B. parkeri*
- 30.: *B. turicatae*
- 31.: *B. crocidurae*
- 32.: *B. anserina*
- 33.: *B. coriaceae*
- 34.: *Treponema pallidum*
- 35.: *Treponema pectinovorum*
- 36.: *Treponema phagedenis*
- 37.: *Leptospira interogans sv. icterohemorrhagiae*
- 38.: *Leptospira inadei sv. lyme*
- 39.: distilled water control
- 40.: 123 Base Pair M. Wt. Marker

### LEGEND TO FIGURE 8

Figure 8 shows the results of amplification from 13 different strains of Borrelia and 5 other strains of spirochetes using sequences derived from the flagellin gene of B. hermsii. The order of strains from left to right in each of the gel pictures 8A-8D is listed above/below.

8A. Agarose gel of the PCR products from 18 bacterial strains after amplification with the B. hermsii primer pair. Amplification is seen only for: B. hermsii, B. parkeri, B. turicatae, B. crocidurae, B. anserina and B. coriaceae. 8B. A Southern blot of the gel shown in Figure 8A which has been probed with an oligonucleotide probe derived from the flagellin gene sequence of B. hermsii.

8C. A Southern blot of the gel shown in Figure 8A which has been probed with the oligonucleotide probe and in 8B but containing two redundancies. 8D. A Southern blot of the gel shown in Figure 8A which has been probed with an oligonucleotide probe derived from the flagellin gene sequence of B. parkeri and B. turicatae.

### INDEX TO LEGEND FOR FIGURE 8

- 1. *: *B. burgdorferi* B31
- 2.: *B. burgdorferi* P/Sto
- 3.: *B. Burgdorferi* P/Bi
- 4.: *B. hermsii* HS1
- 5.: *B. hermsii* Frogner
- 6.: *B. hermsii* YOR-1
- 7.: *B. hermsii* CON-1
- 8.: *B. hermsii* MAN-1
- 9.: *B. parkeri*
- 10.: *B. turicatae*
- 11.: *B. crocidurae*
- 12.: *B. anserina*
- 13.: *B. coriaceae*
- 14.: *B. pallidum*
- 15.: *T. phagedenis*
- 16.: *T. denticola*
- 17.: *L. interogans sv. icterohemorrhagiae*
- 18.: *L. inadei sv. lyme*
- 19.: distilled water control
- 20.: 123 Base Pair M. Wt. Marker

## Claims (Claims for the following Contracting State(s): AT, BE, CH, LI, DE, DK, FR, GB, IT, LU, NL, SE)

1. Primers of complementary strand specificity flanking a sequence of Borrelia burgdorferi to be amplified by nucleic amplification techniques, said primers comprising
a first oligonucleotide selected from the group consisting of sequences comprising about 8-32 substantially consecutive bases from nucleotide numbers 390 to 770 of the flagellin gene open reading frame substantially as described in Figure I or wherein the said flagellin gene is substantially the same as the flagellin gene of B. burgdorferi strain B31 ATCC 35210, and
a second oligonucleotide selected from the group consisting of sequences on the complementary strand comprising about 8-32 substantially consecutive bases having not greater than 70 percent homology with the corresponding sequence of B. hermsii substantially as described in Figure I or wherein the said corresponding sequence is substantially the same as the sequence of the flagellin gene of B. hermsii ATCC 35209.

2. Primers according to Claim I wherein the second oligonucleotide is located spacedly at least 35 nucleotides from the 5' end of the first said primer.

3. An oligonucleotide tracer for detection of Borrelia burgdorferi target sequences comprising
an oligonucleotide probe sequence substantially homologous to a sequence of the interprimer region defined by the first and second oligonucleotides of Claim 1 of the flagellin gene open reading frame of B. burgdorferi, but not greater than 70 percent homologous to the corresponding sequence of B. hermsii, and
signal means conjugated to said probe.

4. A bioassay for detection of Borrelia burgdorferi in a biological sample comprising
extracting the nucleic acid fraction,
denaturing the nucleic acids in the presence of primers of complementary strand specificity to sequences flanking a semiconserved region of nucleic acid encoding the flagellin open reading frame from the region of nucleotide numbers 390 to 770 substantially as described in Figure 1 or wherein the said flagellin gene is substantially the same as the flagellin gene of B. burgdorferi strain B31 ATCC 35210,
hybridizing said primers to said complementary strand sequences,
amplifying the interprimer sequence by nucleic acid amplification techniques,
hybridizing the amplified interprimer sequence to an oligonucleotide tracer comprising a probe having a sequence substantially homologous to a corresponding sequence of the flagellin open reading frame of B. burgdorferi from the region of nucleotide numbers 390 to 770 substantially as described in Figure 1 or wherein the said flagellin gene is substantially the same as the flagellin gene of B. burgdorferi strain B31 ATCC 35210 but not greater than about 70 percent homologous to the corresponding sequence of B. hermsii substantially as described in Figure 1 or wherein the said corresponding sequence is substantially the same as the sequence of the flagellin gene of B. hermsii ATCC 35209, and signal generating means conjugated thereto, and
detecting a signal emitted by said signal-generating means conjugated to said hybridized tracer.

5. A substantially homogeneous assay for detection of Borrelia burgdorferi nucleic acid sequences comprising
denaturing the nucleic acid sequences in the presence of primers of complementary strand specificity to sequences flanking a semiconserved region of nucleic acid encoding the flagellar open reading frame from the region of nucleotide numbers 390 to 770 substantially as described in Figure 1 or wherein the said flagellin gene is substantially the same as the flagellin gene of B. burgdorferi strain B31 ATCC 35210,
hybridizing said primers to said complementary strand sequences,
amplifying the interprimer sequence by nucleic acid amplification techniques,
hybridizing the amplified interprimer sequence to an oligonucleotide tracer comprising a probe having a sequence substantially homologous to a corresponding sequence of the flagellin open reading frame of B. burgdorferi from the region of nucleotide numbers 390 to 770 substantially as described in Figure I or wherein the said flagellin gene is substantially the same as the flagellin gene of B. burgdorferi strain B31 ATCC 35210 but not greater than about 70 percent homologous to the corresponding sequence of B. hermsii substantially as described in Figure 1 or wherein the said corresponding sequence is substantially the same as the sequence of the flagellin gene of B. hermsii ATCC 35209, and a fluorescent tag conjugated thereto, and
measuring the increase in fluorescence polarization.

6. A bioassay for detection of Borrelia burgdorferi or B. hermsii in a biological sample comprising
extracting the nucleic acid fraction,
denaturing the nucleic acid in the presence of primers as defined in Claim 1, 2, 10 or 11
hybridizing said primers to said complementary strand sequences,
amplifying the interprimer sequence by nucleic acid amplification techniques,
and detecting the product of the said amplification.

7. A variant sequence of the Borrelia burgdorferi flagellin gene open reading frame hypervariable region extending from nucleotide number 517 to number 742 substantially as described in Figure 1 or wherein the said flagellin gene is substantially the same as the flagellin gene of B. burgdorferi strain B31 ATCC 35210 comprising base substitutions of a guanosine at positions 531, 549, 552, 612, 613, 684, 693, adenosine at positions 539, 591, 603, 622, 639, 651, 666, thymidine at positions 540, 573, 630, 696, and cytosine at position 735.

8. A variant sequence of the Borrelia burgdorferi flagellin gene open reading frame hypervariable region extending from nucleotide number 517 to number 742 substantially as described in Figure 1 or wherein the said flagellin gene is substantially the same as the flagellin gene of B. burgdorferi strain B31 ATCC 35210 comprising base substitutions of a guanosine at positions 531, 595, 612, 649, and 663, adenosine at positions 539, 552, 651, 666, 670, and 688, thymidine at positions 540, 571, 594, 630, and 696, cytosine at positions 644 and 726.

9. An oligonucleotide tracer for detection of subgroups of Borrelia burgdorferi comprising
an oligonucleotide probe having a sequence selected from the group consisting of 
CTC TGG TGA GGG AGC TCA AAC TGC TCA GGC TGC
ACC GGT TCA AGA GGG T, CTC TGG TGA AGG AGC TCA
GGC TGC TCA GAC TGC ACC TGT TCA AGA AGG, and
TGC TGG TGA GGG AGC TCA AGC TGC TCA GGC TGC
ACC TGT TCA AGA GGG TGC, and
 signal means conjugated to said probe.

10. Primers of complementary strand specificity flanking a sequence of Borrelia hermsii to be amplified by nucleic acid amplification techniques, said primers comprising
a first oligonucleotide selected from the group consisting of sequences comprising about 8-32 substantially consecutive bases from nucleotide numbers 390 to 770 of the flagellin gene open reading frame substantially as described in Figure 1 or wherein the said flagellin gene is substantially the same as the flagellin gene of B. hermsii ATCC 35209, and
a second oligonucleotide selected from the group consisting of sequences on the complementary strand comprising about 8-32 substantially consecutive bases having not greater than 70 percent homology with the corresponding sequence of B. burgdorferi substantially as described in Figure 1 or wherein the said corresponding sequence is substantially the same as the sequence of the flagellin gene of B. burgdorferi strain B31 ATCC 35210.

11. Primers according to Claim 10 wherein the second oligonucleotide is located spacedly at least 35 nucleotides from the 5' end of the first said primer.

12. An oligonucleotide tracer for specific detection of Borrelia hermsii comprising
an oligonucleotide probe having a core sequence extending from nucleotide 606 to 633 of the B. hermsii flagellin gene open reading frame substantially as d escribed in Figure I or wherein the said flagellin gene is substantially the same as the flagellin gene of B. hermsii ATCC 35209, and
signal-generating means.

## Claims (Claims for the following Contracting State(s): ES)

1. A method of making primers of complementary strand specificity flanking a sequence of Borrelia burgdorferi to be amplified by nucleic amplification techniques, said primers comprising
a first oligonucleotide selected from the group consisting of sequences comprising about 8-32 substantially consecutive bases from nucleotide numbers 390 to 770 of the flagellin gene open reading frame substantially as described in Figure 1 or wherein the said flagellin gene is substantially the same as the flagellin gene of B. burgdorferi strain B31 ATCC 35210, and
a second oligonucleotide selected from the group consisting of sequences on the complementary strand comprising about 8-32 substantially consecutive bases having not greater than 70 percent homology with the corresponding sequence of B. hermsii substantially as described in Figure 1 or wherein the said corresponding sequence is substantially the same as the sequence of the flagellin gene of B. hermsii ATCC 35209 comprising synthesising the said primers.

2. A method of making primers according to Claim I wherein the second oligonucleotide is located spacedly at least 35 nucleotides from the 5' end of the first said primer.

3. A method of making an oligonucleotide tracer for detection of Borrelia burgdorferi target sequences comprising
Synthesising an oligonucleotide probe sequence substantially homologous to a sequence of the interprimer region defined by the first and second oligonucleotides of Claim 1 of the flagellin gene open reading frame of B. burgdorferi, but not greater than 70 percent homologous to the corresponding sequence of B. hermsii, and
conjugating signal means to said probe.

4. A bioassay for detection of Borrelia burgdorferi in a biological sample comprising
extracting the nucleic acid fraction,
denaturing the nucleic acids in the presence of primers of complementary strand specificity to sequences flanking a semiconserved region of nucleic acid encoding the flagellin open reading frame from the region of nucleotide numbers 390 to 770 substantially as described in Figure 1 or wherein the said flagellin gene is substantially the same as the flagellin gene of B. burgdorferi strain B31 ATCC 35210,
hybridizing said primers to said complementary strand sequences,
amplifying the interprimer sequence by nucleic acid amplification techniques,
hybridizing the amplified interprimer sequence to an oligonucleotide tracer comprising a probe having a sequence substantially homologous to a corresponding sequence of the flagellin open reading frame of B. burgdorferi from the region of nucleotide numbers 390 to 770 substantially as described in Figure 1 or wherein the said flagellin gene is substantially the same as the flagellin gene of B. burgdorferi strain B31 ATCC 35210 but not greater than about 70 percent homologous to the corresponding sequence of B. hermsii substantially as described in Figure 1 or wherein the said corresponding sequence is substantially the same as the sequence of the flagellin gene of B. hermsii ATCC 35209, and signal generating means conjugated thereto, and
detecting a signal emitted by said signal-generating means conjugated to said hybridized tracer.

5. A substantially homogeneous assay for detection of Borrelia burgdorferi nucleic acid sequences comprising
denaturing the nucleic acid sequences in the presence of primers of complementary strand specificity to sequences flanking a semiconserved region of nucleic acid encoding the flagellar open reading frame from the region of nucleotide numbers 390 to 770 substantially as described in Figure 1 or wherein the said flagellin gene is substantially the same as the flagellin gene of B. burgdorferi strain B31 ATCC 35210,
hybridizing said primers to said complementary strand sequences,
amplifying the interprimer sequence by nucleic acid amplification techniques,
hybridizing the amplified interprimer sequence to an oligonucleotide tracer comprising a probe having a sequence substantially homologous to a corresponding sequence of the flagellin open reading frame of B. burgdorferi from the region of nucleotide numbers 390 to 770 substantially as described in Figure 1 or wherein the said flagellin gene is substantially the same as the flagellin gene of B. burgdorferi strain B31 ATCC 35210 but not greater than about 70 percent homologous to the corresponding sequence of B. hermsii substantially as described in Figure 1 or wherein the said corresponding sequence is substantially the same as the sequence of the flagellin gene of B. hermsii ATCC 35209, and a fluorescent tag conjugated thereto, and
measuring the increase in fluorescence polarization.

6. A bioassay for detection of Borrelia burgdorferi or B. hermsii in a biological sample comprising
extracting the nucleic acid fraction,
denaturing the nucleic acid in the presence of primers as defined in Claim 1, 2, 10 or 11
hybridizing said primers to said complementary strand sequences,
amplifying the interprimer sequence by nucleic acid amplification techniques,
and detecting the product of the said amplification.

7. A method of making a variant sequence of the Borrelia burgdorferi flagellin gene open reading frame hypervariable region extending from nucleotide number 517 to number 742 substantially as described in Figure 1 or wherein the said flagellin gene is substantially the same as the flagellin gene of B. burgdorferi strain B31 ATCC 35210 comprising base substitutions of a guanosine at positions 531, 549, 552, 612, 613, 684, 693, adenosine at positions 539, 591, 603, 622, 639, 651, 666, thymidine at positions 540, 573, 630, 696, and cytosine at position 735 comprising synthesising or isolating said sequence.

8. A method of making a variant sequence of the Borrelia burgdorferi flagellin gene open reading frame hypervariable region extending from nucleotide number 517 to number 742 substantially as described in Figure 1 or wherein the said flagellin gene is substantially the same as the flagellin gene of B. burgdorferi strain B31 ATCC 35210 comprising base substitutions of a guanosine at positions 531, 595, 612, 649, and 663, adenosine at positions 539, 552, 651, 666, 670, and 688, thymidine at positions 540, 571, 594, 630, and 696, cytosine at positions 644 and 726 comprising synthesising or isolating said sequence.

9. A method of making an oligonucleotide tracer for detection of subgroups of Borrelia burgdorferi comprising
Synthesising an oligonucleotide probe having a sequence selected from the group consisting of 
CTC TGG TGA GGG AGC TCA AAC TGC TCA GGC TGC
ACC GGT TCA AGA GGG T, CTC TGG TGA AGG AGC TCA
GGC TGC TCA GAC TGC ACC TGT TCA AGA AGG, and
 
TGC TGG TGA GGG AGC TCA AGC TGC TCA GGC TGC
ACC TGT TCA AGA GGG TGC, and conjugating
 signal means to said probe.

10. A method of making primers of complementary strand specificity flanking a sequence of Borrelia hermsii to be amplified by nucleic acid amplification techniques, said primers comprising
a first oligonucleotide selected from the group consisting of sequences comprising about 8-32 substantially consecutive bases from nucleotide numbers 390 to 770 of the flagellin gene open reading frame substantially as described in Figure 1 or wherein the said flagellin gene is substantially the same as the flagellin gene of B. hermsii ATCC 35209, and
a second oligonucleotide selected from the group consisting of sequences on the complementary strand comprising about 8-32 substantially consecutive bases having not greater than 70 percent homology with the corresponding sequence of B. burgdorferi substantially as described in Figure 1 or wherein the said corresponding sequence is substantially the same as the sequence of the flagellin gene of B. burgdorferi strain B31 ATCC 35210.

11. A method of making primers according to Claim 10 wherein the second oligonucleotide is located spacedly at least 35 nucleotides from the 5' end of the first said primer comprising synthesising the said primers.

12. A method of making an oligonucleotide tracer for specific detection of Borrelia hermsii comprising
Synthesising an oligonucleotide probe having a core sequence extending from nucleotide 606 to 633 of the B. hermsii flagellin gene open reading frame substantially as d escribed in Figure 1 or wherein the said flagellin gene is substantially the same as the flagellin gene of B. hermsii ATCC 35209, and conjugating
signal-generating means to said probe.

## Patentansprüche (Patentansprüche für folgende(n) Vertragsstaat(en): AT, BE, CH, LI, DE, DK, FR, GB, IT, LU, NL, SE)

1. Primer mit komplementärer Strang-Spezifität, die eine Sequenz von Borrelia burgdorferi flankieren, die durch Nucleinsäure-Amplifikationstechniken amplifiziert werden soll, wobei die Primer folgendes aufweisen:
ein erstes Oligonucleotid, das aus der Gruppe ausgewählt ist, die aus Sequenzen besteht, die ca. 8-32 im wesentlichen konsekutive Basen der Nucleotid-Nummern 390 bis 770 des offenen Leserasters des Flagellin-Gens im wesentlichen wie in Figur 1 beschrieben aufweisen, oder wobei das Flagellin-Gen im wesentlichen das gleiche wie das Flagellin-Gen des Stamms B31 ATCC 35210 von B. burgdorferi ist, und
ein zweites Oligonucleotid, das aus der Gruppe ausgewählt ist, die aus Sequenzen an dem komplementären Strang besteht, der ca. 8-32 im wesentlichen konsekutive Basen aufweist, die eine Homologie von nicht mehr als 70 % mit der entsprechenden Sequenz von B. hermsii im wesentlichen wie in Figur 1 beschrieben haben, oder wobei die genannte entsprechende Sequenz im wesentlichen die gleiche wie die Sequenz des Flagellin-Gens von B. hermsii ATCC 35209 ist.

2. Primer nach Anspruch 1, wobei das zweite Oligonucleotid in einem Abstand von wenigstens 35 Nucleotiden von dem 5'-Ende des ersten genannten Primers liegt.

3. Oligonucleotid-Markierung zum Nachweis von Borrelia burgdorferi-Zielsequenzen, wobei die Markierung folgendes aufweist:
eine Oligonucleotidsonden-Sequenz, die im wesentlichen homolog mit einer Sequenz des Interprimer-Bereichs ist,
der durch das erste und das zweite Oligonucleotid von Anspruch 1 des offenen Leserasters des Flagellin-Gens von B. burgdorferi definiert ist, aber eine Homologie von nicht mehr als 70 % mit der entsprechenden Sequenz von B. hermsii hat, und
an die Sonde konjugierte Signalmittel.

4. Bioassay zum Nachweis von Borrelia burgdorferi in einer biologischen Probe, wobei der Bioassay folgendes aufweist:
Extrahieren der Nucleinsäurefraktion,
Denaturieren der Nucleinsäuren in Anwesenheit von Primern mit komplementärer Strangspezifität für Sequenzen, die einen semikonservierten Bereich von Nucleinsäure flankieren, der für den offenen Flagellin-Leseraster von dem Bereich der Nucleotid-Nummern 390 bis 770 im wesentlichen wie in Figur 1 beschrieben codiert, oder wobei das Flagellin-Gen im wesentlichen das gleiche wie das Flagellin-Gen des Stamms B31 ATCC 35210 von B. burgdorferi ist,
Hybridisieren der Primer an die komplementären Strangsequenzen,
Amplifizieren der Interprimer-Sequenz durch Nucleinsäure-Amplifikationstechniken,
Hybridisieren der amplifizierten Interprimer-Sequenz an eine Oligonucleotid-Markierung, die aufweist: eine Sonde, die eine Sequenz hat, die im wesentlichen homolog mit einer entsprechenden Sequenz des offenen Flagellin-Leserasters von B. burgdorferi aus dem Bereich der Nucleotid-Nummern 390 bis 770 im wesentlichen wie in Figur 1 beschrieben ist, oder wobei das Flagellin-Gen im wesentlichen das gleiche wie das Flagellin-Gen vom Stamm B31 ATCC 35210 von B. burgdorferi, aber nicht mehr als ca. 70 % homolog mit der entsprechenden Sequenz von B. hermsii im wesentlichen wie in Figur 1 beschrieben ist, oder wobei die genannte entsprechende Sequenz im wesentlichen die gleiche wie die Sequenz des Flagellin-Gens von B. hermsii ATCC 35209 ist, und daran konjugierte Signalerzeugungsmittel, und
Detektieren eines Signals, das von den an die hybridisierte Markierung konjugierten Signalerzeugungsmitteln ausgesandt wird.

5. Im wesentlichen homogener Assay zum Nachweis von Nucleinsäuresequenzen von Borrelia burgdorferi, wobei der Assay folgendes aufweist:
Denaturieren der Nucleinsäuresequenzen in Anwesenheit von Primern mit komplementärer Strangspezifität für Sequenzen, die einen semikonservierten Nucleinsäurebereich flankieren, der für den flagellaren offenen Leseraster des Bereichs der Nucleotid-Nummern 390 bis 770 im wesentlichen wie in Figur 1 beschrieben codiert, oder wobei das Flagellin-Gen im wesentlichen das gleiche wie das Flagellin-Gen vom Stamm B31 ATCC 35210 von B. burgdorferi ist,
Hybridisieren der Primer an die komplementären Strangsequenzen,
Amplifizieren der Interprimer-Sequenz durch Nucleinsäure-Amplifikationstechniken,
Hybridisieren der amplifizierten Interprimer-Sequenz an eine Oligonucleotid-Markierung, die aufweist: eine Sonde, die eine Sequenz hat, die im wesentlichen homolog mit einer entsprechenden Sequenz des offenen Flagellin-Leserasters von B. burgdorferi aus dem Bereich der Nucleotid-Nummern 390 bis 770 im wesentlichen wie in Figur 1 beschrieben ist, oder wobei das Flagellin-Gen im wesentlichen das gleiche wie das Flagellin-Gen vom Stamm B31 ATCC 35210 von B. burgdorferi, aber nicht mehr als ca. 70 % homolog mit der entsprechenden Sequenz von B. hermsii im wesentlichen wie in Figur 1 beschrieben ist, oder wobei die genannte entsprechende Sequenz im wesentlichen die gleiche wie die Sequenz des Flagellin-Gens von B. hermsii ATCC 35209 ist, und eine daran konjugierte Fluoreszenz-Markierung, und Messen des Anstiegs der Fluoreszenz-Polarisation.

6. Bioassay zum Nachweis von Borrelia burgdorferi oder B. hermsii in einer biologischen Probe, wobei der Bioassay folgendes aufweist:
Extrahieren der Nucleinsäurefraktion,
Denaturieren der Nucleinsäure in Anwesenheit von Primern gemäß der Definition in Anspruch 1, 2, 10 oder 11,
Hybridisieren dieser Primer an die genannten komplementären Strangsequenzen,
Amplifizieren der Interprimer-Sequenz durch Nucleinsäure-Amplifikationstechniken,
und Detektieren des Produkts dieser Amplifikation.

7. Varianten-Sequenz des hypervariablen Bereichs des offenen Leserasters des Flagellin-Gens von Borrelia burgdorferi, der sich von den Nucleotid-Nummern 517 bis 742 im wesentlichen wie in Figur 1 beschrieben erstreckt, oder wobei das Flagellin-Gen im wesentlichen das gleiche wie das Flagellin-Gen des Stamms B31 ATCC 35210 von B. burgdorferi ist, das einen Basenaustausch eines Guanosins an den Positionen 531, 549, 552, 612, 613, 684, 693, von Adenosin an den Positionen 539, 591, 603, 622, 639, 651, 666, von Thymidin an den Positionen 540, 573, 630, 696 und von Cytosin an der Position 735 aufweist.

8. Varianten-Sequenz des hypervariablen Bereichs des offenen Leserasters des Flagellin-Gens von Borrelia burgdorferi, der sich von den Nucleotid Nummern 517 bis 742 im wesentlichen wie in Figur 1 beschrieben erstreckt, oder wobei das Flagellin-Gen im wesentlichen das gleiche wie das Flagellin-Gen des Stamms B31 ATCC 35210 von B. burgdorferi ist, das einen Basenaustausch eines Guanosins an den Positionen 531, 595, 612, 649 und 663, von Adenosin an den Positionen 539, 552, 651, 666, 670 und 688, von Thymidin an den Positionen 540, 571, 594, 630 und 696, von Cytosin an den Positionen 644 und 726 aufweist.

9. Oligonucleotid-Markierung zum Nachweis von Untergruppen von Borrelia burgdorferi, wobei die Markierung folgendes aufweist:
eine Oligonucleotid-Sonde, die eine Sequenz hat, die aus der Gruppe ausgewählt ist, die aus den folgenden besteht: 
CTC TGG TGA GGG AGC TCA AAC TGC TCA GGC TGC
ACC GGT TCA AGA GGG T, CTC TGG TGA AGG AGC TCA
GGC TGC TCA GAC TGC ACC TGT TCA AGA AGG, und
TGC TGG TGA GGG AGC TCA AGC TGC TCA GGC TGC
ACC TGT TCA AGA GGG TGC, und
 an diese Sonde konjugierte Signalmittel.

10. Primer mit komplementärer Strangspezifität, die eine Sequenz von Borrelia hermsii flankieren, die durch Nucleinsäure-Amplifikationstechniken zu amplifizieren ist, wobei diese Primer folgendes aufweisen:
ein erstes Oligonucleotid, das aus der Gruppe ausgewählt ist, die aus Sequenzen besteht, die ungefähr 8-32 im wesentlichen konsekutive Basen der Nucleotid-Nummern 390 bis 770 des offenen Leserasters des Flagellin-Gens im wesentlichen wie in Figur 1 beschrieben aufweisen, oder wobei das Flagellin-Gen im wesentlichen das gleiche wie das Flagellin-Gen von B. hermsii ATCC 35209 ist, und
ein zweites Oligonucleotid, das aus der Gruppe ausgewählt ist, die aus Sequenzen an dem komplementären Strang bestehen, der ungefähr 8-32 im wesentlichen konsekutive Basen aufweist, die eine Homologie von nicht mehr als 70 % mit der entsprechenden Sequenz von B. burgdorferi haben, im wesentlichen wie in Figur 1 beschrieben aufweisen, oder wobei die genannte entsprechende Sequenz im wesentlichen die gleiche wie die Sequenz des Flagellin-Gens des Stamms B31 ATCC 35210 von B. burgdorferi ist.

11. Primer nach Anspruch 10, wobei das zweite Oligonucleotid um wenigstens 35 Nucleotide im Abstand von dem 5'-Ende des ersten genannten Primers liegt.

12. Oligonucleotid-Markierung zum spezifischen Nachweis von Borrelia hermsii, die folgendes aufweist: eine Oligonucleotid-Sonde, die eine Kernsequenz hat, die sich von Nucleotid 603 bis 633 des offenen Leserasters des Flagellin-Gens von B. hermsii im wesentlichen wie in Figur 1 beschrieben erstreckt, oder wobei das Flagellin-Gen im wesentlichen das gleiche wie das Flagellin-Gen von B. hermsii ATCC 35209 ist, und Signalerzeugungsmittel.

## Patentansprüche (Patentansprüche für folgende(n) Vertragsstaat(en): ES)

1. Verfahren zum Herstellen von Primern mit komplementärer Strang-Spezifität, die eine Sequenz von Borrelia burgdorferi flankieren, die durch Nucleinsäure-Amplifikationstechniken amplifiziert werden soll, wobei die Primer folgendes aufweisen:
ein erstes Oligonucleotid, das aus der Gruppe ausgewählt ist, die aus Sequenzen besteht, die ca. 8-32 im wesentlichen konsekutive Basen der Nucleotid-Nummern 390 bis 770 des offenen Leserasters des Flagellin-Gens im wesentlichen wie in Figur 1 beschrieben aufweisen, oder wobei das Flagellin-Gen im wesentlichen das gleiche wie das Flagellin-Gen des Stamms B31 ATCC 35210 von B. burgdorferi ist, und
ein zweites Oligonucleotid, das aus der Gruppe ausgewählt ist, die aus Sequenzen an dem komplementären Strang besteht, der ca. 8-32 im wesentlichen konsekutive Basen aufweist, die eine Homologie von nicht mehr als 70 % mit der entsprechenden Sequenz von B. hermsii im wesentlichen wie in Figur 1 beschrieben haben, oder wobei die genannte entsprechende Sequenz im wesentlichen die gleiche wie die Sequenz des Flagellin-Gens von B. hermsii ATCC 35209 ist.

2. Verfahren zum Herstellen von Primern nach Anspruch 1, wobei das zweite Oligonucleotid in einem Abstand von wenigstens 35 Nucleotiden von dem 5'-Ende des ersten genannten Primers liegt.

3. Verfahren zum Herstellen einer Oligonucleotid-Markierung zum Nachweis von Borrelia burgdorferi-Zielsequenzen, wobei das Verfahren folgendes aufweist:
Synthetisieren einer Oligonucleotidsonden-Sequenz, die im wesentlichen homolog mit einer Sequenz des Interprimer-Bereichs ist, der durch das erste und das zweite Oligonucleotid von Anspruch 1 des offenen Leserasters des Flagellin-Gens von B. burgdorferi definiert ist, aber eine Homologie von nicht mehr als 70 % mit der entsprechenden Sequenz von B. hermsii hat, und Konjugieren von Signalmitteln an die Sonde.

4. Bioassay zum Nachweis von Borrelia burgdorferi in einer biologischen Probe, wobei der Bioassay folgendes aufweist:
Extrahieren der Nucleinsäurefraktion,
Denaturieren der Nucleinsäuren in Anwesenheit von Primern mit komplementärer Strangspezifität für Sequenzen, die einen semikonservierten Bereich von Nucleinsäure flankieren, der für den offenen Flagellin-Leseraster von dem Bereich der Nucleotid-Nummern 390 bis 770 im wesentlichen wie in Figur 1 beschrieben codiert, oder wobei das Flagellin-Gen im wesentlichen das gleiche wie das Flagellin-Gen des Stamms B31 ATCC 35210 von B. burgdorferi ist,
Hybridisieren der Primer an die komplementären Strangsequenzen,
Amplifizieren der Interprimer-Sequenz durch Nucleinsäure-Amplifikationstechniken,
Hybridisieren der amplifizierten Interprimer-Sequenz an eine Oligonucleotid-Markierung, die aufweist: eine Sonde, die eine Sequenz hat, die im wesentlichen homolog mit einer entsprechenden Sequenz des offenen Flagellin-Leserasters von B. burgdorferi aus dem Bereich der Nucleotid-Nummern 390 bis 770 im wesentlichen wie in Figur 1 beschrieben ist, oder wobei das Flagellin-Gen im wesentlichen das gleiche wie das Flagellin-Gen vom Stamm B31 ATCC 35210 von B. burgdorferi, aber nicht mehr als ca. 70 % homolog mit der entsprechenden Sequenz von B. hermsii im wesentlichen wie in Figur 1 beschrieben ist, oder wobei die genannte entsprechende Sequenz im wesentlichen die gleiche wie die Sequenz des Flagellin-Gens von B. hermsii ATCC 35209 ist, und daran konjugierte Signalerzeugungsmittel, und Detektieren eines Signals, das von den an die hybridisierte Markierung konjugierten Signalerzeugungsmitteln ausgesandt wird.

5. Im wesentlichen homogener Assay zum Nachweis von Nucleinsäuresequenzen von Borrelia burgdorferi, wobei der Assay folgendes aufweist:
Denaturieren der Nucleinsäuresequenzen in Anwesenheit von Primern mit komplementärer Strangspezifität für Sequenzen, die einen semikonservierten Nucleinsäurebereich flankieren, der für den flagellaren offenen Leseraster des Bereichs der Nucleotid-Nummern 390 bis 770 im wesentlichen wie in Figur 1 beschrieben codiert, oder wobei das Flagellin-Gen im wesentlichen das gleiche wie das Flagellin-Gen vom Stamm B31 ATCC 35210 von B. burgdorferi ist,
Hybridisieren der Primer an die komplementären Strangsequenzen,
Amplifizieren der Interprimer-Sequenz durch Nucleinsäure-Amplifikationstechniken,
Hybridisieren der amplifizierten Interprimer-Sequenz an eine Oligonucleotid-Markierung, die aufweist: eine Sonde, die eine Sequenz hat, die im wesentlichen homolog mit einer entsprechenden Sequenz des offenen Flagellin-Leserasters von B. burgdorferi aus dem Bereich der Nucleotid-Nummern 390 bis 770 im wesentlichen wie in Figur 1 beschrieben ist, oder wobei das Flagellin-Gen im wesentlichen das gleiche wie das Flagellin-Gen vom Stamm B31 ATCC 35210 von B. burgdorferi, aber nicht mehr als ca. 70 % homolog mit der entsprechenden Sequenz von B. hermsii im wesentlichen wie in Figur 1 beschrieben ist, oder wobei die genannte entsprechende Sequenz im wesentlichen die gleiche wie die Sequenz des Flagellin-Gens von B. hermsii ATCC 35209 ist, und eine daran konjugierte Fluoreszenz-Markierung, und Messen des Anstiegs der Fluoreszenz-Polarisation.

6. Bioassay zum Nachweis von Borrelia burgdorferi oder B. hermsii in einer biologischen Probe, wobei der Bioassay folgendes aufweist:
Extrahieren der Nucleinsäurefraktion,
Denaturieren der Nucleinsäure in Anwesenheit von Primern gemäß der Definition in Anspruch 1, 2, 10 oder 11,
Hybridisieren dieser Primer an die genannten komplementären Strangsequenzen,
Amplifizieren der Interprimer-Sequenz durch Nucleinsäure-Amplifikationstechniken,
und Detektieren des Produkts dieser Amplifikation.

7. Verfahren zum Herstellen einer Varianten-Sequenz des hypervariablen Bereichs des offenen Leserasters des Flagellin-Gens von Borrelia burgdorferi, der sich von den Nucleotid-Nummern 517 bis 742 im wesentlichen wie in Figur 1 beschrieben erstreckt, oder wobei das Flagellin-Gen im wesentlichen das gleiche wie das Flagellin-Gen des Stamms B31 ATCC 35210 von B. burgdorferi ist, das einen Basenaustausch eines Guanosins an den Positionen 531, 549, 552, 612, 613, 684, 693, von Adenosin an den Positionen 539, 591, 603, 622, 639, 651, 666, von Thymidin an den Positionen 540, 573, 630, 696 und von Cytosin an der Position 735 aufweist, wobei das Verfahren die Synthetisierung oder Isolierung der genannten Sequenz aufweist.

8. Verfahren zum Herstellen einer Varianten-Sequenz des hypervariablen Bereichs des offenen Leserasters des Flagellin-Gens von Borrelia burgdorferi, der sich von den Nucleotid-Nummern 517 bis 742 im wesentlichen wie in Figur 1 beschrieben erstreckt, oder wobei das Flagellin-Gen im wesentlichen das gleiche wie das Flagellin-Gen des Stamms B31 ATCC 35210 von B. burgdorferi ist, das einen Basenaustausch eines Guanosins an den Positionen 531, 595, 612, 649 und 663, von Adenosin an den Positionen 539, 552, 651, 666, 670 und 688, von Thymidin an den Positionen 540, 571, 594, 630 und 696, von Cytosin an den Positionen 644 und 726 aufweist, wobei das Verfahren die Synthetisierung oder Isolierung der genannten Sequenz aufweist.

9. Verfahren zum Herstellen einer Oligonucleotid-Markierung zum Nachweis von Untergruppen von Borrelia burgdorferi, wobei das Verfahren folgendes aufweist: Synthetisieren einer Oligonucleotid-Sonde, die eine Sequenz hat, die aus der Gruppe ausgewählt ist, die aus den folgenden besteht: 
CTC TGG TGA GGG AGC TCA AAC TGC TCA GGC TGC
ACC GGT TCA AGA GGG T, CTC TGG TGA AGG AGC TCA
GGC TGC TCA GAC TGC ACC TGT TCA AGA AGG, und
TGC TGG TGA GGG AGC TCA AGC TGC TCA GGC TGC
ACC TGT TCA AGA GGG TGC, und
 Konjugieren von Signalmitteln an diese Sonde.

10. Verfahren zum Herstellen von Primern mit komplementärer Strangspezifität, die eine Sequenz von Borrelia hermsii flankieren, die durch Nucleinsäure-Amplifikationstechniken zu amplifizieren ist, wobei diese Primer folgendes aufweisen:
ein erstes Oligonucleotid, das aus der Gruppe ausgewählt ist, die aus Sequenzen besteht, die ungefähr 8-32 im wesentlichen konsekutive Basen der Nucleotid-Nummern 390 bis 770 des offenen Leserasters des Flagellin-Gens im wesentlichen wie in Figur 1 beschrieben aufweisen, oder wobei das Flagellin-Gen im wesentlichen das gleiche wie das Flagellin-Gen von B. hermsii ATCC 35209 ist, und
ein zweites Oligonucleotid, das aus der Gruppe ausgewählt ist, die aus Sequenzen an dem komplementären Strang bestehen, der ungefähr 8-32 im wesentlichen konsekutive Basen aufweist, die eine Homologie von nicht mehr als 70 % mit der entsprechenden Sequenz von B. burgdorferi haben, im wesentlichen wie in Figur 1 beschrieben aufweisen, oder wobei die genannte entsprechende Sequenz im wesentlichen die gleiche wie die Sequenz des Flagellin-Gens des Stamms B31 ATCC 35210 von B. burgdorferi ist.

11. Verfahren zum Herstellen von Primern nach Anspruch 10, wobei das zweite Oligonucleotid um wenigstens 35 Nucleotide im Abstand von dem 5'-Ende des ersten genannten Primers liegt, wobei das Verfahren die Synthetisierung dieser Primer aufweist.

12. Verfahren zum Herstellen einer Oligonucleotid-Markierung zum spezifischen Nachweis von Borrelia hermsii, das folgendes aufweist:
Synthetisieren einer Oligonucleotid-Sonde, die eine Kernsequenz hat, die sich von Nucleotid 603 bis 633 des offenen Leserasters des Flagellin-Gens von B. hermsii im wesentlichen wie in Figur 1 beschrieben erstreckt, oder wobei das Flagellin-Gen im wesentlichen das gleiche wie das Flagellin-Gen von B. hermsii ATCC 35209 ist, und
Konjugieren von Signalerzeugungsmitteln an diese Sonde.

## Revendications (Revendications pour l'(les) Etat(s) contractant(s) suivant(s): AT, BE, CH, LI, DE, DK, FR, GB, IT, LU, NL, SE)

1. Amorces présentant une spécificité de brin complémentaire situées à côté d'une séquence de Borrelia burgdorferi destinée à être amplifiée par des techniques d'amplification nucléique, lesdites amorces comprenant :
un premier oligonucléotide choisi parmi l'ensemble comprenant les séquences comprenant d'environ 8 à 32 bases essentiellement consécutives, allant du nucléotide N° 390 au nucléotide N° 770 du cadre ouvert de lecture du gène de la flagelline, essentiellement comme décrit dans la Figure 1, ou encore où ledit gène de la flagelline est essentiellement le même que le gène de la flagelline de la souche de B. burgdorferi B31 ATCC 35210, et
un deuxième oligonucléotide, choisi parmi l'ensemble comprenant les séquences du brin complémentaire, comprenant d'environ 8 à 32 bases essentiellement consécutives ayant une homologie non supérieure à 70 % avec la séquence correspondante de B. hermsii, essentiellement comme décrit sur la Figure 1, ou encore où ladite séquence correspondante est essentiellement la même que la séquence du gène de la flagelline de B. hermsii ATCC 35209.

2. Amorces selon la revendication 1, dans lesquelles le deuxième oligonucléotide est situé à une distance d'au moins 35 nucléotides de l'extrémité 5' de ladite première amorce.

3. Traceur oligonucléotidique, pour détecter les séquences cibles de Borrelia burgdorferi, comprenant
une séquence de sonde oligonucléotidique essentiellement homologue d'une séquence de la région située entre les amorces, définie par le premier et le deuxième oligo-nucléotides de la revendication 1, du cadre ouvert de lecture du gène de la flagelline de B. burgdorferi, mais dont l'homologie n'est pas supérieure à 70 % avec la séquence correspondante de B. hermsii, et
des moyens de signaux, conjugués de ladite sonde.

4. Analyse biologique pour détecter Borrelia burgdorferi dans un échantillon biologique, qui consiste :
- à extraire la fraction d'acides nucléiques,
- à dénaturer les acides nucléiques en présence d'amorces ayant une spécificité de brin complémentaire vis-à-vis de séquences situées à côté d'une région semi-conservée d'un acide nucléique codant le cadre ouvert de lecture de la flagelline, dans la région comprise entre le nucléotide N° 390 et le nucléotide N° 770, essentiellement comme décrit sur la Figure 1, ou encore où ledit gène de la flagelline est essentiellement le même que le gène de la flagelline de la souche de B. burgdorferi B31 ATCC 35210,
à hybrider lesdites amorces auxdites séquences de brin complémentaire,
à amplifier la séquence entre amorces par des techniques d'amplification des acides nucléiques,
à hybrider la séquence amplifier entre amorces sur un traceur oligonucléotidique comprenant une sonde ayant une séquence essentiellement homologue à une séquence correspondante du cadre ouvert de lecture de la flagelline de B. burgdorferi, dans la région allant du nucléotide N° 390 au nucléotide N° 770, essentiellement comme décrit sur la Figure 1, ou encore où ledit gène de la flagelline est essentiellement le même que le gène de la flagelline de la souche de B. burgdorferi B31 ATCC 35210 ou présente une homologie non supérieure à environ 70 % avec la séquence correspondante de B. hermsii, essentiellement comme décrit sur la Figure 1, ou encore où ladite séquence correspondante est essentiellement la même que la séquence du gène de la flagelline de B. hermsii ATCC 35209, ainsi que des moyens générateurs de signaux, qui lui sont conjugués, et
à détecter un signal émis par lesdits moyens générateurs de signaux, conjugués audit traceur hybridé.

5. Analyse essentiellement homogène, pour détecter les séquences d'acides nucléiques de Borrelia burgdorferi, qui consiste :
à dénaturer les séquences d'acides nucléiques, en présence d'amorces ayant une spécificité de brin complémentaire vis-à-vis de séquences situées à côté d'une région semi-conservée d'un acide nucléique codant le cadre ouvert de lecture flagellaire, dans la région allant du nucléotide N° 390 au nucléotide N° 770, essentiellement comme décrit sur la Figure 1, ou encore où ledit gène de la flagelline est essentiellement le même que la gène de la flagelline de la souche de B. burgdorferi B31 ATCC 35210,
à hybrider lesdites amorces auxdites séquences de brin complémentaire,
à amplifier la séquence entre amorces par des techniques d'amplification des acides nucléiques,
à hybrider la séquence amplifier entre amorces sur un traceur oligonucléotidique comprenant une sonde ayant une séquence essentiellement homologue à une séquence correspondante du cadre ouvert de lecture de la flagelline de B. burgdorferi, dans la région allant du nucléotide N° 390 au nucléotide N° 770, essentiellement comme décrit sur la Figure 1, ou encore où ledit gène de la flagelline est essentiellement le même que le gène de la flagelline de la souche de B. burgdorferi B31 ATCC 35210 ou présente une homologie non supérieure à environ 70 % avec la séquence correspondante de B. hermsii, essentiellement comme décrit sur la Figure 1, ou encore où ladite séquence correspondante est essentiellement la même que la séquence du gène de la flagelline de B. hermsii ATCC 35209, et une étiquette fluorescente qui lui est conjuguée, et
à mesurer l'augmentation de la polarisation en fluorescence.

6. Analyse biologique pour détecter Borrelia burgdorferi ou B. hermsii dans un échantillon biologique, qui consiste
à extraire la fraction d'acide nucléique,
à dénaturer l'acide nucléique en présence d'amorces selon les revendications 1, 2, 10 ou 11,
à hybrider lesdites amorces sur lesdites séquences de brins complémentaires,
à amplifier la séquence entre amorces par des techniques d'amplification des acides nucléiques, et à détecter le produit de ladite amplification.

7. Séquence variante de la région hypervariable du cadre ouvert de lecture du gène de la flagelline de Borrelia burgdorferi, s'étendant du nucléotide N° 517 au nucléotide N° 742, essentiellement comme décrit sur la Figure 1, ou encore où ledit gène de la flagelline est essentiellement le même que le gène de la flagelline de la souche de B. burgdorferi B31 ATCC 35210 comprenant des substitutions de bases d'une guanosine sur les positions 531, 549, 552, 612, 613, 684, 693, d'une adénosine sur les positions 539, 591, 603, 622, 639, 651, 666, d'une thymidine sur les positions 540, 573, 630, 696 et d'une cytosine sur la position 735.

8. Séquence variante de la région hypervariable du cadre ouvert de lecture du gène de la flagelline de Borrelia burgdorferi, s'étendant du nucléotide N° 517 au nucléotide N° 742, essentiellement comme décrit sur la Figure 1, ou encore où ledit gène de la flagelline est essentiellement le même que le gène de la flagelline de la souche de B. burgdorferi B31 ATCC 35210 comprenant des substitutions de bases d'une guanosine sur les positions 531, 595, 612, 649 et 663, d'une adénosine sur les positions 539, 552, 651, 666, 670 et 688, d'une thymidine sur les positions 540, 571, 630 et 696, et d'une cytosine sur les positions 644 et 726.

9. Traceur oligonucléotidique pour détecter des sous-groupes de Borrelia burgdorferi, comprenant
une sonde oligonucléotidique ayant une séquence choisie parmi l'ensemble comprenant 
CTC TGG TGA GGG AGC TCA AAC TGC TCA GGC TGC
ACC GGT TCA AGA GGG T,CTC TGG TGA AGG AGC TCA
GGC TGC TCA GAC TGC ACC TGT TCA AGA AGG, et
CTC TGG TGA GGG AGC TCA AAC TGC TCA GGC TGC
ACC TGT TCA AGA GGG TGC
 et des moyens de signaux conjugués de ladite sonde.

10. Amorces présentant une spécificité de brin complémentaire situées à côté d'une séquence de Borrelia hermsii destinée à être amplifiée par des techniques d'amplification nucléique, lesdites amorces comprenant :
un premier oligonucléotide choisi parmi l'ensemble comprenant les séquences comprenant d'environ 8 à 32 bases essentiellement consécutives, allant du nucléotide N° 390 au nucléotide N° 770 du cadre ouvert de lecture du gène de la flagelline, essentiellement comme décrit dans la Figure 1, ou encore où ledit gène de la flagelline est essentiellement le même que le gène de la flagelline de B. hermsii ATCC 35209, et
un deuxième oligonucléotide, choisi parmi l'ensemble comprenant les séquences du brin complémentaire, comprenant d'environ 8 à 32 bases essentiellement consécutives ayant une homologie non supérieure à 70 % avec la séquence correspondante de B. hermsii, essentiellement comme décrit sur la Figure 1, ou encore où ladite séquence correspondante est essentiellement la même que la séquence du gène de la flagelline de la souche de B. burgdorferi B31 ATCC 35210.

11. Amorces selon la revendication 10, dans lesquelles le deuxième oligonucléotide est situé à une distance d'au moins 35 nucléotides de l'extrémité 5' de ladite première amorce.

12. Traceur oligonucléotidique pour la détection spécifique de Borrelia hermsii, comprenant
une sonde oligonucléotidique ayant une séquence de coeur s'étendant du nucléotide N° 606 au nucléotide N° 633 du cadre ouvert de lecture du gène de la flagelline de B. hermsii, essentiellement comme décrit sur la Figure 1, ou encore où ledit gène de la flagelline est essentiellement le même que le gène de la flagelline de B. hermsii ATCC 35209, et
des moyens générateurs de signaux.

## Revendications (Revendications pour l'(les) Etat(s) contractant(s) suivant(s): ES)

1. Procédé pour préparer des amorces présentant une spécificité de brin complémentaire situées à côté d'une séquence de Borrelia burgdorferi destinée à être amplifiée par des techniques d'amplification nucléique, lesdites amorces comprenant :
un premier oligonucléotide choisi parmi l'ensemble comprenant les séquences comprenant d'environ 8 à 32 bases essentiellement consécutives, allant du nucléotide N° 390 au nucléotide N° 770 du cadre ouvert de lecture du gène de la flagelline, essentiellement comme décrit dans la Figure 1, ou encore où ledit gène de la flagelline est essentiellement le même que le gène de la flagelline de la souche de B. burgdorferi B31 ATCC 35210, et
un deuxième oligonucléotide, choisi parmi l'ensemble comprenant les séquences du brin complémentaire, comprenant d'environ 8 à 32 bases essentiellement consécutives ayant une homologie non supérieure à 70 % avec la séquence correspondante de B. hermsii, essentiellement comme décrit sur la Figure 1, ou encore où ladite séquence correspondante est essentiellement la même que la séquence du gène de la flagelline de B. hermsii ATCC 35209 qui consiste à synthétiser lesdites amorces.

2. Procédé pour préparer des amorces selon la revendication 1, dans lesquelles le deuxième oligonucléotide est situé à une distance d'au moins 35 nucléotides de l'extrémité 5' de ladite première amorce.

3. Procédé pour préparer un traceur oligonucléotidique, pour détecter les séquences cibles de Borrelia burgdorferi, qui consiste
a synthétiser une séquence de sonde oligonucléotidique essentiellement homologue d'une séquence de la région située entre les amorces, définie par le premier et le deuxième oligonucléotides de la revendication 1, du cadre ouvert de lecture du gène de la flagelline de B. burgdorferi, mais dont l'homologie n'est pas supérieure à 70 % avec la séquence correspondante de B. hermsii, et
à conjuguer un moyen de signaux à ladite sonde.

4. Analyse biologique pour détecter Borrelia burgdorferi dans un échantillon biologique, qui consiste :
- à extraire la fraction d'acides nucléiques,
- à dénaturer les acides nucléiques en présence d'amorces ayant une spécificité de brin complémentaire vis-à-vis de séquences situées à côté d'une région semi-conservée d'un acide nucléique codant le cadre ouvert de lecture de la flagelline, dans la région comprise entre le nucléotide N° 390 et le nucléotide N° 770, essentiellement comme décrit sur la Figure 1, ou encore où ledit gène de la flagelline est essentiellement le même que le gène de la flagelline de la souche de B. burgdorferi B31 ATCC 35210,
à hybrider lesdites amorces auxdites séquences de brin complémentaire,
à amplifier la séquence entre amorces par des techniques d'amplification des acides nucléiques,
à hybrider la séquence amplifier entre amorces sur un traceur oligonucléotidique comprenant une sonde ayant une séquence essentiellement homologue à une séquence correspondante du cadre ouvert de lecture de la flagelline de B. burgdorferi, dans la région allant du nucléotide N° 390 au nucléotide N° 770, essentiellement comme décrit sur la Figure 1, ou encore où ledit gène de la flagelline est essentiellement le même que le gène de la flagelline de la souche de B. burgdorferi B31 ATCC 35210 ou présente une homologie non supérieure à environ 70 % avec la séquence correspondante de B. hermsii, essentiellement comme décrit sur la Figure 1, ou encore où ladite séquence correspondante est essentiellement la même que la séquence du gène de la flagelline de B. hermsii ATCC 35209, ainsi que des moyens générateurs de signaux, qui lui sont conjugués, et
à détecter un signal émis par lesdits moyens générateurs de signaux, conjugués audit traceur hybridé.

5. Analyse essentiellement homogène, pour détecter les séquences d'acides nucléiques de Borrelia burgdorferi, qui consiste :
à dénaturer les séquences d'acides nucléiques, en présence d'amorces ayant une spécificité de brin complémentaire vis-à-vis de séquences situées à côté d'une région semi-conservée d'un acide nucléique codant le cadre ouvert de lecture flagellaire, dans la région allant du nucléotide N° 390 au nucléotide N° 770, essentiellement comme décrit sur la Figure 1, ou encore où ledit gène de la flagelline est essentiellement le même que la gène de la flagelline de la souche de B. burgdorferi B31 ATCC 35210,
à hybrider lesdites amorces auxdites séquences de brin complémentaire,
à amplifier la séquence entre amorces par des techniques d'amplification des acides nucléiques,
à hybrider la séquence amplifier entre amorces sur un traceur oligonucléotidique comprenant une sonde ayant une séquence essentiellement homologue à une séquence correspondante du cadre ouvert de lecture de la flagelline de B. burgdorferi, dans la région allant du nucléotide N° 390 au nucléotide N° 770, essentiellement comme décrit sur la Figure 1, ou encore où ledit gène de la flagelline est essentiellement le même que le gène de la flagelline de la souche de B. burgdorferi B31 ATCC 35210 ou présente une homologie non supérieure à environ 70 % avec la séquence correspondante de B. hermsii, essentiellement comme décrit sur la Figure 1, ou encore où ladite séquence correspondante est essentiellement la même que la séquence du gène de la flagelline de B. hermsii ATCC 35209, et une étiquette fluorescente qui lui est conjuguée, et
à mesurer l'augmentation de la polarisation en fluorescence.

6. Analyse biologique pour détecter Borrelia burgdorferi ou B. hermsii dans un échantillon biologique, qui consiste
à extraire la fraction d'acide nucléique,
à dénaturer l'acide nucléique en présence d'amorces selon les revendications 1, 2, 10 ou 11,
à hybrider lesdites amorces sur lesdites séquences de brins complémentaires,
à amplifier la séquence entre amorces par des techniques d'amplification des acides nucléiques,
et à détecter le produit de ladite amplification.

7. Procédé pour préparer une séquence variante de la région hypervariable du cadre ouvert de lecture du gène de la flagelline de Borrelia burgdorferi, s'étendant du nucléotide N° 517 au nucléotide N° 742, essentiellement comme décrit sur la Figure 1, ou encore où ledit gène de la flagelline est essentiellement le même que le gène de la flagelline de la souche de B. burgdorferi B31 ATCC 35210 comprenant des substitutions de bases d'une guanosine sur les positions 531, 549, 552, 612, 613, 684, 693, d'une adénosine sur les positions 539, 591, 603, 622, 639, 651, 666, d'une thymidine sur les positions 540, 573, 630, 696 et d'une cytosine sur la position 735 qui consiste à synthétiser ou à isoler ladite séquence.

8. Procédé pour préparer une séquence variante de la région hypervariable du cadre ouvert de lecture du gène de la flagelline de Borrelia burgdorferi, s'étendant du nucléotide N" 517 au nucléotide N° 742, essentiellement comme décrit sur la Figure 1, ou encore où ledit gène de la flagelline est essentiellement le même que le gène de la flagelline de la souche de B. burgdorferi B31 ATCC 35210 comprenant des substitutions de bases d'une guanosine sur les positions 531, 595, 612, 649 et 663, d'une adénosine sur les positions 539, 552, 651, 666, 670 et 688, d'une thymidine sur les positions 540, 571, 630 et 696, et d'une cytosine sur les positions 644 et 726 qui consiste à synthétiser ou isoler ladite séquence.

9. Procédé pour préparer un traceur oligonucléotidique pour détecter des sous-groupes de Borrelia burgdorferi, qui consiste
à synthétiser une sonde oligonucléotidique ayant une séquence choisie parmi l'ensemble comprenant 
CTC TGG TGA GGG AGC TCA AAC TGC TCA GGC TGC
ACC GGT TCA AGA GGG T,CTC TGG TGA AGG AGC TCA
GGC TGC TCA GAC TGC ACC TGT TCA AGA AGG, et
CTC TGG TGA GGG AGC TCA AAC TGC TCA GGC TGC
ACC TGT TCA AGA GGG TGC
 et à conjuguer lesdits moyens de signaux conjugués à ladite sonde.

10. Procédé pour préparer des amorces présentant une spécificité de brin complémentaire situées à côté d'une séquence de Borrelia hermsii destinée à être amplifiée par des techniques d'amplification nucléique, lesdites amorces comprenant :
un premier oligonucléotide choisi parmi l'ensemble comprenant les séquences comprenant d'environ 8 à 32 bases essentiellement consécutives, allant du nucléotide N° 390 au nucléotide N° 770 du cadre ouvert de lecture du gène de la flagelline, essentiellement comme décrit dans la Figure 1, ou encore où ledit gène de la flagelline est essentiellement le même que le gène de la flagelline de B. hermsii ATCC 35209, et
un deuxième oligonucléotide, choisi parmi l'ensemble comprenant les séquences du brin complémentaire, comprenant d'environ 8 à 32 bases essentiellement consécutives ayant une homologie non supérieure à 70 % avec la séquence correspondante de B. hermsii, essentiellement comme décrit sur la Figure 1, ou encore où ladite séquence correspondante est essentiellement la même que la séquence du gène de la flagelline de la souche de B. burgdorferi B31 ATCC 35210.

11. Procédé pour préparer des amorces selon la revendication 10, dans lesquelles le deuxième oligonucléotide est situé à une distance d'au moins 35 nucléotides de l'extrémité 5' de ladite première amorce.

12. Procédé pour préparer un traceur oligonucléotidique pour la détection spécifique de Borrelia hermsii, qui consiste
à synthétiser une sonde oligonucléotidique ayant une séquence de coeur s'étendant du nucléotide N° 606 au nucléotide N° 633 du cadre ouvert de lecture du gène de la flagelline de B. hermsii, essentiellement comme décrit sur la Figure 1, ou encore où ledit gène de la flagelline est essentiellement le même que le gène de la flagelline de B. hermsii ATCC 35209, et
à conjuguer lesdits moyens générateurs de signaux à ladite sonde.
